(19) Europäisches Patentamt
European Patent Office
Office européen des brevets

(11) EP 3 366 296 B1

(12) # EUROPEAN PATENT SPECIFICATION

(45) Date of publication and mention of the grant of the patent:
**15.01.2020 Bulletin 2020/03**

(21) Application number: **16856981.2**

(22) Date of filing: **24.10.2016**

(51) Int Cl.:
***A61K 31/704*** *(2006.01)* ***A61P 1/00*** *(2006.01)*

(86) International application number:
**PCT/ES2016/070753**

(87) International publication number:
**WO 2017/068227 (27.04.2017 Gazette 2017/17)**

(54) **METHODS FOR USING REGULATORS FOR INCREASING THE EXPRESSION OR ACTIVATION OF P53 AND/OR REGULATORS FOR REDUCING OR INHIBITING THE EXPRESSION OF P63-ALPHA, FOR THE TREATMENT OF NON-ALCOHOLIC FATTY LIVER DISEASE (NAFLD) AND/OR NON-ALCOHOLIC STEATOHEPATITIS (NASH)**

VERFAHREN ZUR VERWENDUNG VON REGULATOREN ZUR ERHÖHUNG DER EXPRESSION ODER AKTIVIERUNG VON P53 UND/ODER REGULATOREN ZUR VERRINGERUNG ODER HEMMUNG DER EXPRESSION VON P63-ALPHA ZUR BEHANDLUNG VON NICHT ALKOHOLISCHER FETTLEBERERKRANKUNG (NAFLD) UND/ODER NICHTALKOHOLISCHER STEATOHEPATITIS (NASH)

MÉTHODES POUR UTILISER DES RÉGULATEURS D'AUGMENTATION DE L'EXPRESSION OU D'ACTIVATION DE P53 ET/OU DES RÉGULATEURS DE RÉDUCTION OU INHIBITEURS DE L'EXPRESSION DE P63-ALPHA POUR LE TRAITEMENT DE NAFLD (STÉATOSE HÉPATIQUE NON ALCOOLIQUE) ET/OU NASH (STÉATOHÉPATITE NON ALCOOLIQUE)

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **22.10.2015 EP 15382519**

(43) Date of publication of application:
**29.08.2018 Bulletin 2018/35**

(73) Proprietor: **Universidade De Santiago De Compostela**
**15782 Santiago de Compostela (La Coruña) (ES)**

(72) Inventors:
- **NOGUEIRAS POZO, Rubén**
  **15782 Santiago de Compostela (La Coruña) (ES)**
- **DIÉGUEZ GONZÁLEZ, Carlos**
  **15782 Santiago de Compostela (La Coruña) (ES)**
- **LOZA GARCÍA, Mabel**
  **15782 Santiago de Compostela (La Coruña) (ES)**

(74) Representative: **Hoffmann Eitle**
**Hoffmann Eitle S.L.U.**
**Paseo de la Castellana 140, 3a planta**
**Edificio LIMA**
**28046 Madrid (ES)**

(56) References cited:
**US-A1- 2005 119 193** **US-A1- 2007 082 856**
**US-A1- 2007 265 213**

- **CASTRO RUI E; FERREIRA DUARTE M S; AFONSO MARTA B; BORRALHO PEDRO M; MACHADO MARIANA V; CORTEZ-PINTO HELENA; RODRIGUES CECÍLIA M P: "TI-miR-34a/SIRT1/p53 is suppressed by ursodeoxycholic acid in the rat liver and activated by disease severity in human non-alcoholic fatty liver disease.AU", J. OF HEPATOLOGY, vol. 28, 15 August 2012 (2012-08-15), - 15 September 1981 (1981-09-15), XP002790792,**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 3 366 296 B1

- KENGO TOMITA ET AL: "p53/p66Shc-mediated signaling contributes to the progression of non-alcoholic steatohepatitis in humans and mice", JOURNAL OF HEPATOLOGY, vol. 57, no. 4, 1 January 2012 (2012-01-01), pages 837-843, XP55461642, AMSTERDAM, NL ISSN: 0168-8278, DOI: 10.1016/j.jhep.2012.05.013
- AMARAL J D ET AL.: 'p53 and the regulation of hepatocyte apoptosis: implications for disease pathogenesis.' TRENDS IN MOLECULAR MEDICINE vol. 15, ISSN 1471-4914 pages 531 - 541, XP026756434

## Description

### Technical field of the invention

The present invention refers to the biotechnological field, more particularly to the use of up-regulators of the expression of p53 and/or down-regulators or inhibitors of the expression of p63 for the treatment of NAFLD (Non-alcoholic fatty liver disease) and/or NASH (non-alcoholic steatohepatitis).

### Background of the invention

The following discussion of the background of the invention is merely provided to aid the reader in understanding the invention, and is not admitted to describe or constitute prior art to the present invention.

NAFLD (Non-alcoholic fatty liver disease) and NASH (non-alcoholic steatohepatitis) are two of the most common liver diseases associated with obesity, type 2 diabetes and the metabolic syndrome. Nevertheless, the fact that some obese patients almost never develop hepatic disease while a few subjects with normal BMI or discrete overweight develop NAFLD and NASH has prompted the search for different genes that may protect or exacerbate the development of the disease in relation to diet.

p53 belongs to a family of transcription factors that also includes p63 and p73, functional homologs of p53 sharing high sequence and structural similarities. The transcription factor p53 is best known for its function as a tumor suppressor. Alterations in metabolism are crucial for tumor progression and tumor cells survival and thereby it is logical that p53 is deeply involved in the control of certain metabolic and cellular dysfunctions. In this regard, one of the key actions of p53 is the regulation of lipid metabolism. In general, p53 inhibits lipid synthesis and induces fatty acid oxidation. However, the link between p53 and hepatic lipid metabolism is currently confuse and controversial. Whereas some reports indicate that p53 is an essential player in the pathogenesis of alcoholic and NAFLD; others suggest that this transcription factor attenuates liver steatosis. The conclusions of those studies were relied on gene expression results, the use of pharmacological compounds, mice lacking p53 globally or *in vitro* assays. However, there are no studies evaluating the physiological relevance of hepatic p53 through the specific manipulation of this transcription factor in liver.

p53 acts as a primary sensor in the cellular response to stress by promoting cell fate decisions and regulating several adaptive responses. The endoplasmic reticulum (ER) is an organelle that plays a crucial role in stress response and cell metabolism. ER-transmembrane-signaling molecules regulate lipid metabolism and as a matter of fact, ER stress has an important role in the development and progression of NAFLD. Therefore, to acquire knowledge on the hepatic role of p53 on NAFLD, it is essential to investigate if the molecular pathways altered after gain- and loss-of function studies on liver p53 might involve changes in ER stress.

In the present study, we demonstrate that p53 null mice develop hepatic steatosis when fed with either chow diet or HFD (high fat diet) before they develop any sign of tumor incidence. Gain- and loss-of-function experiments using viral particles inhibiting or activating p53 specifically in the liver led to impaired and ameliorated hepatic steatosis, steatohepatitis and ER stress respectively. Moreover, the hepatic levels of p63 were inversely correlated to hepatic p53. Consistently, the inhibition of hepatic p63 attenuated the liver condition of mice lacking p53 in the liver, indicating that p63 mediates the hepatic actions of p53.

In addition, in the present study we demonstrate that one of such agents capable of up-regulating the expression of p53 in the hepatocyte cells of a human subject and thus useful for the present invention, is the chemical compound known as doxorubicin as well as analogues thereof.

We thus herein propose a novel strategy for the treatment of non-alcoholic fatty acid disease (NAFLD) and/or non-alcoholic steatohepatitis (NASH) which includes the repositioning of the pharmaceutical drug doxorubicin.

Document D1 (Castro Rui E et al; "TI-miR-34a/SIRT1/p53 is suppressed by ursodeoxycholic acid in the rat liver and activated by disease severity in human non-alcoholic fatty liver disease.AU", J. OF HEPATOLOGY, vol. 28, 15 August 2012 (2012-08-15), 0 15 September 1981), and document D2 (kengo Tomita et al; "p53/p66hc-mediated signalling contributes to the progression of non-alcoholic steatohepatitis in humans and mice", Journal of Hepatology, vol. 57, no. 4, 1 January 2012, pages 837-843), disclose that the p53 pathway may represent a target for the treatment of NASH. However, neither of these documents actually shows an agent being used in such manner.

### Brief description of the invention

In summary, the results shown herein demonstrate that an up-regulator of the expression of the p53 protein in hepatocyte cells and/or a down-regulator or inhibitor of the expression of the p63 protein in hepatocyte cells is useful for the production of a medicament for use in the treatment of non-alcoholic fatty acid disease (NAFLD) and/or non-alcoholic steatohepatitis (NASH). In particular, one of such agents capable of up-regulating the expression of p53 in the hepatocyte cells of a human subject and thus useful for the present invention, is the chemical compound known as

doxorubicin. The present invention demonstrates the usefulness of this drug by illustrating a series of experiments in which 0.3, 0.6, 1.25, 2.5 and 5 mg/kg of doxorubicin were administered intra-peritoneally to mice of the Swiss strain fed with a standardize diet. In these experiments it can be observed how the latter mentioned dosages provoked a significant loss of weight as shown in figure 1A in a dose dependent manner, wherein preferably dosages greater or equal to 0.6 mg/kg reduced the body weight of the mice significantly (see Figures 1A and 1B).

Therefore, the results shown herein demonstrate that increasing of intracellular p53 concentration and/or the decreasing of intracellular p63 reduces hepatic steatosis. There was no way to infer, prior to the findings disclosed herein, that increased concentrations of intracellular p53 and/or decreased concentrations of intracellular p63 would have reduced hepatic steatosis. The results disclosed herein are thus the first to allow this interpretation.

## Brief description of the drawings

The accompanying drawings, which are incorporated in and constitute a part of this specification, illustrate several embodiments and together with the description illustrate the disclosed compositions and methods.

**Figure 1.** Effect of the administration of doxorubicin (0.3, 0.6, 1.25, 2.5 and 5 mg/kg intraperitoneal 1 injection/day) during 5 days on body weight change (A) and cumulative food intake (B) of adult male Swiss mice. *$p<0.05$, ***$p<0.001$, n=7-8 per group.

**Figure 2.** Effect of the administration of doxorubicin (0.15, 0.3, 0.6 and 1.25 mg/kg intraperitoneal twice per week) during 68 days on body weight change (A), cumulative food intake (B), body composition (C), serum troponin levels (D) of adult male Swiss mice. *$p<0.05$, ***$p<0.001$, n=7-8 per group.

**Figure 3.** Effect of the administration of doxorubicin (0.6 mg/kg intraperitoneal twice per week) during 68 days on liver morphology (A) and (B), hepatic triglyceride content, hepatic non-esterified fatty acids and hepatic cholesterol levels (C), liver mRNA levels of the inflammatory markers TNF$\alpha$, Arginase, IL6, NOS2 and F480 (D) and liver protein levels of FAS, LPL, CPT1, FGF21, pIRE, XBP1s, CHOP, cleaved caspase 3, pJNK/JNK, ACC, pACC of adult male Swiss mice. *$p<0.05$, ***$p<0.001$, n=7-8 per group.

**Figure 4.** Effect of the administration of doxorubicin (0.15, 0.3, and 0.6 mg/kg intraperitoneal twice per week) during 60 days on body weight change (A) and cumulative food intake (B) of adult male C57/B6 mice. *$p<0.05$, **$p<0.01$, n=7-8 per group and (C) hepatic TAG (mg/gr).

**Figure 5.** This figure (figure 5A to 5D) shows a series of experiments in which a vehicle, quercetin 15 mg/kg, quercetin 15mg/Kg + ADR 20mg/Kg and quercetin 15mg/Kg + ADR 10mg/Kg were administered orally twice per week during a period of 30 days to mice of the C57/B6 strain fed with a HFD (60% total fat content) during a period of two months. In these experiments, it can be observed how the latter mentioned dosages (quercetin 15mg/Kg + ADR 20mg/Kg and quercetin 15mg/Kg + ADR 10mg/Kg) provoked a reduction in the body weight and a reduction in hepatic tryglicerides, particularly in the case of quercetin 15mg/Kg + ADR 20mg/Kg.

**Figure 6.** Metabolic phenotype of male p53 deficient mice fed a chow diet or high fat diet. (A) Body weight of male mice after free access to chow diet or high fat diet. (B) Cumulative food intake over 24h. (C) Fat mass and non-fat mass. (D) Glucose tolerance test in male p53 null mice fed a chow diet ot high fat diet. (E) Insulin tolerance test in male p53 null mice fed a chow diet ot high fat diet. *$p<0.05$, **$p<0.01$, n=7-8 per group.

**Figure 7.** Effect of p53 deficiency on liver steatosis and steatohepatitis of mice fed a chow diet or high fat diet. (A) Hematoxylin eosin (upper panel) and oil red staining (lower panel). (B) Total liver triglyceride (TG), serum AST and ALT. (C) mRNA expression of PPAR$\gamma$, LPL, SCARB1, MTTP, PGC1$\alpha$, TNF$\alpha$, IL6, F480, arginase, NOS2, MAC2 in the liver of WT and p53 null mice. Hepatic LPL activity of WT and p53 null mice. (D) Representative western blot of protein levels of FAS, LPL, pJNK/JNK, pIRE/IRE, Xbp1, pPERK, pEIF2$\alpha$/eIF2$\alpha$, caspase 3, cleaved caspase 3, caspase 7 and cleaved caspase 7. Comparison between WT and p53 null mice were analyzed in the same gel. Dividing lines indicate splicings in the same gel. *$p<0.05$, **$p<0.01$, n=7-8 per group.

**Figure 8.** Effect of liver p53 silencing on liver steatosis and steatohepatitis. (A) Efficiency of the down-regulation of p53 in the liver after the injection in tail vein of associate adenovirus serotype 8 (AAV8) expressing Cre in p53floxed mice. (B) Hematoxylin eosin (upper panel) and oil red staining (lower panel). (C) Total liver triglyceride (TG) content, serum AST and ALT. (D) Representative western blot of protein levels of FAS, LPL, pJNK/JNK, pIRE/IRE, Xbp1, pPERK, pEIF2$\alpha$/eIF2$\alpha$, cleaved caspase 3 and cleaved caspase 7. Dividing lines indicate splicings in the same gel.

Hepatic LPL activity (E) Glucose tolerance test and (F) insulin tolerance test. *p<0.05, **p<0.01, n=7-8 per group.

**Figure 9.** Effect of p53 silencing in HepG2 cells. (A) Efficiency of the down-regulation of p53 in HepG2 cells transfected with adenoviruses expressing GFP alone or adenoviruses encoding a p53 negative dominant. (B) Oil red staining in HepG2 cells treated with etoposide at different concentrations. (C) Representative western blot of protein levels of peIF2$\alpha$ and Xbp1 in HepG2 cells non-treated and treated with etoposide. *p<0.05, n=4 per group.

**Figure 10.** Hepatic rescue of p53 in mice fed a high fat diet ameliorates p53-induced liver steatosis and steatohepatitis. (A) GFP protein levels in the liver and BAT of WT and p53 null mice after the tail vein injection of adenoviruses encoding either GFP or p53. (B) p53 expression in the liver of WT and p53 null mice after the injection of adenoviruses encoding either GFP or p53. (C) Total liver triglyceride (TG) content. (D) serum AST. (E) Hematoxylin eosin (upper panel) and oil red staining (lower panel). (F) Representative western blot of protein levels of FAS, LPL, pJNK/JNK, pIRE/IRE, Xbp1, pPERK, pEIF2$\alpha$/eIF2$\alpha$, cleaved caspase 3 and cleaved caspase 7 in the liver of p53 null mice after the injection of adenoviruses encoding either GFP or p53. Dividing lines indicate splicings in the same gel. *p<0.05, **p<0.01, n=7-8 per group.

**Figure 11.** Hepatic p63 mediates the actions of p53 in the liver. Protein levels of p63 in the liver of (A) wild type and p53 null mice; (B) control mice and mice lacking p53 in the liver; (C) wild type and p53 null mice after the injection of adenoviruses encoding either GFP or p53. (D) Efficiency of the down-regulation of p63 in the liver after the injection in tail vein of lentiviruses shp63. (E) Hematoxylin eosin (upper panel) and oil red staining (lower panel). (F) Total liver triglyceride (TG) content, serum AST and ALT. (G) Representative western blot of protein levels of FAS, LPL, pJNK/JNK, pIRE/IRE, Xbp1, pPERK, pEIF2$\alpha$/eIF2$\alpha$, cleaved caspase 3 and cleaved caspase 7. Dividing lines indicate splicings in the same gel. Hepatic LPL activity. *p<0.05, **p<0.01, n=7-8 per group.

**Figure 12.** This figure shows how the doxorubicin protects the accumulation of lipids induced by oleic acid in human hepatocytes (see examples).

**Description of the invention**

List of abbreviations

**NAFLD:** Non-alcoholic fatty liver disease
**NASH:** non-alcoholic steatohepatitis
**WAT:** white adipose tissue
**TG:** triglycerides
**NEFAs:** non-esterified fatty acids
**ACC:** acetyl-CoA carboxylase
**FAS:** fatty acid synthase
**PPARy:** peroxisome proliferator-activated receptor gamma
**SCARB1:** scavenger receptor class B type 1
**LPL:** lipoprotein lipase
**ALT:** alanine aminotransferase
**AST:** aspartate aminotransferase
**XBP1:** X-box binding protein 1
**PERK:** protein kinase RNA-like ER kinase

Definitions

As used in the specification and the appended claims the term "p53 protein" also known as p53, cellular tumor antigen p53 (UniProt name), phosphoprotein p53, tumor suppressor p53, antigen NY-CO-13, or transformation-related protein 53 (TRP53), must be understood as any isoform of a protein encoded by homologous genes in various organisms, such as TP53 (humans) and Trp53 (mice). The p53 protein is crucial in multicellular organisms, where it regulates the cell cycle and, thus, functions as a tumor suppressor, preventing cancer. As such, p53 has been described as "the guardian of the genome" because of its role in conserving stability by preventing genome mutation. Hence TP53 is classified as a tumor suppressor gene. The name p53 is in reference to its apparent molecular mass: SDS-PAGE analysis indicates that it is a 53-kilodalton (kDa) protein. However, based on calculations from its amino acid residues, p53's

mass is actually only 43.7 kDa. This difference is due to the high number of proline residues in the protein; these slow its migration on SDS-PAGE, thus making it appear heavier than it actually is. This effect is observed with p53 from a variety of species, including humans, rodents, frogs, and fish.

As used in the specification and the appended claims the nucleotide sequence/s for "p63" can be taken from the GenBank database (http://www.ncbi.nlm.nih.gov/Genbank/). A variant of any of these sequences, based on the identity of the total length of the nucleotide sequence, having at least 80%, 85%, 90%, 95%, 97%, 98% or 99 % is also included in the present invention.

As used in the specification and the appended claims the term "up-regulator" must be understood as a compound capable of increasing the intracellular concentration of the p53 protein in the hepatocytes of a subject relative to that observed in the absence of the compound.

As used in the specification and the appended claim the term "down-regulator" must be understood as a compound capable of decreasing the intracellular concentration of the p63 protein in the hepatocytes of a subject relative to that observed in the absence of the compound.

The up-regulator or activator agent can be a modulator of the intracellular expression of p53 that increases the amount of p53. In addition the activator agent can be a compound capable of increasing the intracellular concentration of p53, or of increasing the concentration of p53-encoding mRNA, and/or the posttranslational modification of p53 (if any).

The activator can either directly or indirectly affect the expression of p53. Activators can be identified, for example, through screening methods as described herein in the detailed description of the invention. An example of an activator compound which induces p53 expression by indirectly affecting the expression of p53 would be an inhibitor of an endogenous inhibitor of p53.

The down-regulator can be a modulator of the intracellular expression of p53 that decreases the amount of p63. In addition the down-regulator can be a compound capable of decreasing the intracellular concentration of p63, or of decreasing the concentration of p63 -encoding mRNA, and/or the posttranslational modification of p63 (if any).

The down-regulator can either directly or indirectly affect the expression of p63. Down-regulators or inhibitors of p63 can be identified, for example, through screening methods as described herein in the detailed description of the invention.

The term "increases" or "increasing" refers to increases above basal level. For example, basal levels are normal in vivo levels prior to, or in the absence of, addition of an activator compound.

The term "decreases" or "decreasing" refers to decreases below basal level. For example, basal levels are normal in vivo levels prior to, or in the absence of, addition of a down-regulator o inhibitor compound.

In this specification and in the claims which follow, reference will be made to a number of terms which shall be defined to have the following meanings:

"Optional" or "optionally" means that the subsequently described event or circumstance may or may not occur, and that the description includes instances where said event or circumstance occurs and instances where it does not.

The term "pharmaceutically acceptable carrier" is intended to include formulation used to stabilize, solubilize and otherwise be mixed with active ingredients to be administered to living animals, including humans. This includes any and all solvents, dispersion media, coatings, antibacterial and antifungal agents, isotonic and absorption delaying agents, and the like, compatible with pharmaceutical administration. Except insofar as any conventional media or agent is incompatible with the active compound, such use in the compositions is contemplated.

The term "disease" as used herein is intended to be generally synonymous, and is used interchangeably with, the terms "disorder" and "condition" (as in medical condition), in that all reflect an abnormal condition of the body or of one of its parts that impairs normal functioning and is typically manifested by distinguishing signs and symptoms.

As used herein, Non-alcoholic fatty liver disease (NAFLD) is one cause of a fatty liver, occurring when fat is deposited (steatosis) in the liver not due to excessive alcohol use. It is related to insulin resistance and the metabolic syndrome and may respond to treatments originally developed for other insulin-resistant states (e.g. diabetes mellitus type 2) such as weight loss, metformin and thiazolidinediones. Non-alcoholic steatohepatitis (NASH) is the most extreme form of NAFLD, and is regarded as a major cause of cirrhosis of the liver of unknown cause.

The term "combination therapy" means the administration of two or more therapeutic agents to treat a therapeutic condition or disorder described in the present disclosure. Such administration encompasses co-administration of these therapeutic agents in a substantially simultaneous manner, such as in a single capsule having a fixed ratio of active ingredients or in multiple, separate capsules for each active ingredient. In addition, such administration also encompasses use of each type of therapeutic agent in a sequential manner. In either case, the treatment regimen will provide beneficial effects of the compound combination in treating the conditions or disorders described herein.

The phrase "therapeutically effective" is intended to qualify the amount of active ingredients used in the treatment of a disease or disorder. This amount will achieve the goal of reducing or eliminating the said disease or disorder.

The term "subject" means all mammals including humans. Examples of subjects includes, but are not limited to, humans, cows, dogs, cats, goats, sheep, pigs, and rabbits.

Throughout this application, various publications are referenced. The disclosures of these publications in their

entireties are hereby incorporated by reference into this application in order to more fully describe the state of the art to which this pertains. The references disclosed are also individually and specifically incorporated by reference herein for the material contained in them that is discussed in the sentence in which the reference is relied upon.

Detailed description of the invention

p53 is an intensively studied protein, primarily as a tumor suppressor in humans and other mammals. p53 mutations or deficiency are highly correlated with increased susceptibility to cancer, and most studies have focused on how p53 might protect malignant progression. However, apart from cell proliferation, p53 also plays an important role in different biological functions including longevity, stress, ageing, and obesity-associated disorders such as hepatic steatosis and insulin resistance.

In this sense, the authors of the present invention have investigated the endogenous metabolic role of p53 in the liver under different diet conditions by using p53 null mice before these mice developed any sign of tumor incidence. Based on these experiments, we can herein conclude for the first time that a down-regulation of endogenous p53 causes liver steatosis, independently of the type of diet. In this sense, we herein show that although p53 null mice gain less weight than their WT counterparts when challenged with a HFD (high-fat-diet) they show an exacerbated liver steatosis.

In addition, the authors of the present invention found higher levels of FAS and LPL in the liver of p53 null mice, suggesting an increased hepatic fatty acid synthesis and uptake. In regard to ER stress, an event that is present in liver of obese rodents and characterizes the metabolic syndrome, protein levels of markers of ER stress were up-regulated in the liver of p53 null mice in comparison to their littermates. Consistent with these findings, the down-regulation of p53 in the liver mimicked the results observed in global p53 null mice, and the lack of p53 in HepG2 cells impaired their response to etoposide, thereby accumulating more lipid droplets than control cells. In agreement with loss of function studies, the rescue of p53 in the liver of p53 null mice attenuated HFD-induced hepatic steatosis by decreasing fatty acid accumulation and ER stress. Taken together, these findings suggest that genetic manipulation of p53 specifically in the liver exerts a profound influence upon liver metabolism, with loss of p53 leading to harmful effects and its activation beneficial for liver condition. The present results are clearly independent of nutritional status, as the mice were fed ad libitum.

As regards the downstream molecular pathways controlling the hepatic actions of p53, the present results clearly show that the global down-regulation or the specific hepatic inhibition of p53 increases the levels of ER stress markers whereas the rescue of p53 in mice lacking this transcription factor ameliorates both ER stress and liver steatosis. Therefore, these results point out to a negative feedback loop between p53 and ER stress in liver.

Furthermore, the authors of the present invention have found that hepatic levels of downstream target genes of p53 such as bax or p66shc, that were reported to regulate lipid metabolism, remained unaltered in the experiments shown herein. However, surprisingly hepatic p63 protein levels were negatively regulated by p53, and more importantly, the down-regulation of p63 in the liver attenuated p53-induced liver ER stress, fatty acid deposition and steatosis. The interaction between p63and p53 is strong and, at least at cellular level, they can supplement their functions each other. The present findings thus indicate that the down-regulation of p63 in the liver attenuates p53-induced liver steatosis and stimulates FAS expression to mediate its pro-survival effects. Thus, our results indicate that p63 also plays a critical role in hepatic lipid metabolism and mediates the hepatic actions of p53.

Overall, the present findings indicate that reduced levels of endogenous p53 in the whole body, and in particular in the liver, leads to increased ER stress and liver steatosis independent of diet, and impair the response of hepatocytes to etoposide. The rescue of hepatic p53 in global p53 null mice is sufficient to attenuate ER stress and liver steatosis. In addition, the actions of p53 on lipid homeostasis in the liver is mediated by p63, as there is a negative correlation between their levels, and the down-regulation of p63 in the liver attenuates p53-induced liver steatosis.

In summary, these results demonstrate that an up-regulator of the expression of the p53 protein in hepatocyte cells and/or a down-regulator or inhibitor of the expression of the p63α protein in hepatocyte cells is useful for the production of a medicament for use in the treatment of non-alcoholic fatty acid disease (NAFLD) and/or non-alcoholic steatohepatitis (NASH).

Thus, these results demonstrate that increasing of intracellular p53 concentration and/or the decreasing of intracellular p63 α reduces hepatic steatosis. There was no way to infer, prior to the findings disclosed herein, that increased concentrations of intracellular p53 and/or decreased concentrations of intracellular p63α would have reduced hepatic steatosis. The results disclosed herein are thus the first to allow this interpretation.

Consequently, p53 and/or p63α are identified herein as crucial targets to provide and develop new compositions suitable as drugs for treating non-alcoholic fatty acid disease (NAFLD) and/or non-alcoholic steatohepatitis (NASH). Therefore, disclosed herein are thus methods of increasing the expression or activity of the p53 protein in the hepatocytes of a subject or methods of decreasing the expression or activity of the p63 in the hepatocytes of a subject for treating non-alcoholic fatty acid disease (NAFLD) and/or non-alcoholic steatohepatitis (NASH), the method comprising: a) identifying a subject who may benefit from p53 expression or p63 reduction; and b) administering to the subject an activator

of p53 and/or a down-regulator of p63.

Consequently, a first aspect of the invention refers to a an agent capable of up-regulating the expression of p53 and/or down-regulating or inhibiting the expression of p63 in the hepatocyte cells of a human subject suffering from non-alcoholic fatty acid disease (NAFLD) or non-alcoholic steatohepatitis (NASH), relative to that observed in the absence of the agent, for use in the treatment of non-alcoholic fatty acid disease (NAFLD) and/or non-alcoholic steatohepatitis (NASH).

One of such agents capable of up-regulating the expression of p53 in the hepatocyte cells of a human subject and thus useful for the present invention, is the chemical compound known as doxorubicin. In the context of the present invention, the term "doxorubicin" trade name Adriamycin; pegylated liposomal form trade name Doxil; nonpegylated liposomal form trade name Myocet, also known as hydroxydaunorubicin and hydroxydaunomycin, is an anthracycline antibiotic closely related to the natural product daunomycin and like all anthracyclines, it works by intercalating DNA, with the most serious adverse effect being life-threatening heart damage. It is commonly used in the treatment of a wide range of cancers, including hematological malignancies (blood cancers, like leukaemia and lymphoma), many types of carcinoma (solid tumours) and soft tissue sarcomas. It is often used in combination chemotherapy as a component of various chemotherapy regimens.

The chemical structure of doxorubicin is as follows:

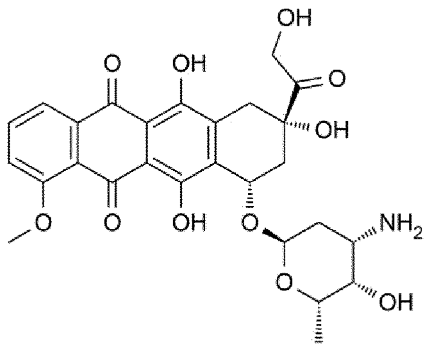

In order to demonstrate the usefulness of this drug, the authors of the present invention have conducted a series of experiments in which 0.3, 0.6, 1.25, 2.5 and 5 mg/kg of doxorubicin were administered intra-peritoneally to mice of the Swiss strain fed with a standardize diet. In these experiments it can be observed how the latter mentioned dosages provoked a significant loss of weight as shown in figure 1A in a dose dependent manner, wherein preferably dosages greater or equal to 0.6 mg/kg reduced the body weight of the mice significantly (see Figures 1A and 1B).

Based on these results, the authors of the present invention conducted a further series of experiments using doxorubicin in an animal model of hepatic steatosis, in particular in an animal model of HFD-induced obese mice. This specific animal model suffers from hepatic steatosis and accurately reproduces human obesity.

In these further series of experiments, as already detailed above, mice were fed with a HFD (45% total fat content) during a period of 12 weeks, and after said period of time, mice were treated intra-peritoneally with 0.15, 0.3, 0.6 and 1.25 mg/kg of doxorubicin during a further period of 2 months. During this two month period, doxorubicin was administered twice per week using each of the different dosages above mentioned.

After said period of two months, we observed a significant loss of body weight at all studied dosages as clearly reflected in Fig. 2A. Moreover, these experiments demonstrate that a dose of 0.6 mg/kg body weight is remarkably reduced without altering the normal food intake of the mice as reflected in Fig. 2B. This latter mentioned result suggests that the use of doxorubicin (at this dosage) does not result in any significant adverse effect, more so when the reduction in body weight has been shown to correspond to a reduce quantity of fat without altering the muscle mass as shown in figure 2C.

In addition, we determined whether the intra-peritoneal administration of dosages of 0,6 mg/kg provoked cardiotoxicity in the animal model. For this purpose, we determined the levels of troponine, a marker of cardiovascular damage, after the administration of the latter mentioned dosage. As shown in figure 2D, doxorubicin at a fixed dose of 0,6 mg/kg, fails to significantly increase the levels of troponine. This result clearly suggests the absence of cardiotoxic effects of doxorubicin at this dose.

Once we have determined that dosages of 0,6 mg/kg fail to result in significant adverse effects in the studied animal model, we conducted a further series of studies specifically directed to the liver. First of all, we conducted a histological study and observed that obese mice treated with doxorubicin at a dosage of 0,6 mg/kg during a period of time of two months have reduced hepatic damage as shown in figure 3A. Additionally, the level of triglycerides in the liver of these mice is also reduced in comparison to animals treated with a control vehicle while the level of non-esterified

fatty acids in their liver is increased (see figure 3B). This suggests that doxorubicin increases the oxidation of fatty acids in the liver.

At a molecular level, we found that treatment with doxorubicin (at a dose of 0,6 mg/kg) during a two month period, reduces the expression of inflammatory markers such as TNF-a, arginase, IL-6, NOS2 and F480 (see figure 3C). This specific type of treatment also reduced the protein levels of genes implicated in ER stress (please note that this type of stress is proportional to the severity of the steatosis) such as pIRE, XBP1, CHOP y pJNK (see figure 3D)

Furthermore, the authors of the present invention in order to corroborate the above mentioned results, conducted a still further series of experiments by using mice of the C57/B6 strain fed with a HFD (60% total fat content) during a period of 12 weeks. Subsequently, mice were administered a dose of 0,6 mg/kg intra-peritoneally twice per week during a further period of two months. The results shown in figures 4A and C indicate that mice reduced their body weight significantly and that hepatic tryglicerides were also significantly reduced.

Moreover, the authors conducted a series of experiments in which a vehicle, quercetin 15 mg/kg, quercetin 15mg/Kg + ADR 20mg/Kg and quercetin 15mg/Kg + ADR 10mg/Kg were administered orally twice per week during a period of 30 days to mice of the C57/B6 strain fed with a HFD (60% total fat content) during a period of two months. In these experiments, as shown in figure 5, it can be observed how the latter mentioned dosages (quercetin 15mg/Kg + ADR 20mg/Kg and quercetin 15mg/Kg + ADR 10mg/Kg) provoked a reduction in the body weight and a reduction in hepatic tryglicerides, particularly in the case of quercetin 15mg/Kg + ADR 20mg/Kg.

Consequently, a preferred embodiment of the first aspect of the invention refers to doxorubicin or a pharmaceutically acceptable salt thereof or a pharmaceutically acceptable vehicle or carrier comprising doxorubicin or a pharmaceutical acceptable salt thereof such as a liposome (doxil or myocet), for use in the treatment of non-alcoholic fatty acid disease (NAFLD) and/or non-alcoholic steatohepatitis (NASH) in a human subject. Preferably, such compositions are administered by any suitable administration route, preferably orally or intravenously and, whenever appropriate, intravesical and intra-arterial routes.

For any administration route suitable for the present invention, dosage is usually calculated on the basis of body surface area (mg/m2) or on the basis of mg/kg. The optimal dose will be selected according to the administration route, treatment regime and/or administration schedule, having regard to the existing toxicity and effectiveness data. It is noted that in adult humans, 100 mg/kg is equivalent to 100/mg/kg x 37 kg/sq.m = 3700 mg/sq.m.

In a preferred embodiment the doxorubicin is in a dosage capable of providing a therapeutic effect in the absence or with minor toxic effects. Accordingly, in the present invention, the dosage of doxorubicin is not particularly restricted. However, preferably doxorubicin may be administered to human beings orally alone or in combination with other active ingredients in dosages up to 5.0 mg/kg/week (185 mg/m2/week), preferably between 0.8 mg/kg/week (29.6 mg/m2/week) and 5.0 mg/kg/week (185 mg/m2/week), more preferably about 3.2 mg/kg/week (120 mg/m2/week). Preferably, the dose-schedule to be delivered of oral forms of doxorubicin is measure for a human adult of about 60 kg of weight.

Doxorubicin may also be administered intravenously and, whenever appropriate, intravesical and intra-arterial routes at a dosage of below 0.80 mg/kg/week (29.6 mg/m2/week), preferably doxorubicin may be administered intravenously, alone or in combination, in dosages below 0.40 mg/kg/week (14.8 mg/m2/week), more preferably below 0.20 mg/kg/week (7.5 mg/m2/week), still more preferably about 0.10 mg/kg/week (3.75 mg/m2/week), still more preferably about 0.05 mg/kg/week (1.85 mg/m2/week). Preferred ranges for the intravenous administration are from about 0.0121 mg/kg/week (0.4477 mg/m2/week) to about 0.80 mg/kg/week (29.6 mg/m2/week), preferably from about 0.024 mg/kg/week (0.9 mg/m2/week) to about 0.20 mg/kg/week (7.5 mg/m2/week) and more preferably from about 0.024 mg/kg/week (0.9 mg/m2/week) to about 0.10 mg/kg/week (3.75 mg/m2/week). Preferably, the dose-schedule to be delivered of intravenous forms of doxorubicin is measure for a human adult of about 60 kg of weight.

The doxorubicin dose-schedule to be delivered may differ depending on its use within a specific regimen (e.g. as a single agent or in combination with other agents such as quercetin or as a part of multidisciplinary approaches which include combination with hormonotherapy). Intravenous administration of doxorubicin should be performed with caution. It is recommended to administer doxorubicin into the tubing of a freely flowing IV infusion (isotonic sodium chloride or 5% glucose solution) over a period of 3 to 5 minutes. This technique is intended to minimize the risk of thrombosis or perivenous extravasation which could lead to severe cellulitis, vesication and tissue necrosis. A direct push injection is not recommended due to the risk of extravasation, which may occur even in the presence of adequate blood return upon needle aspiration. Intravenous administration of doxorubicin may be preferably performed every week, every two weeks or every 20 o 21 days.

In addition, said pharmaceutical compositions comprising doxorubicin are formulated to be compatible with its intended route of administration. Methods to accomplish the administration are known to those of ordinary skill in the art.

In another preferred embodiment of the first aspect of the invention, said agent is characterized by being capable of up-regulating the expression of p53 and down-regulating or inhibiting the expression of p63$\alpha$ in the hepatocyte cells of a human subject suffering from non-alcoholic fatty acid disease (NAFLD) and/or non-alcoholic steatohepatitis (NASH) relative to that observed in the absence of the agent.

As already mentioned in the definitions above, activator compounds, such as doxorubicin, are thus those

molecules that increased p53 functional activity or alter its intracellular distribution. In one embodiment, a compound is an activator compound when the compound reduces the incidence, severity or adverse consequences of non-alcoholic fatty acid disease (NAFLD) and/or non-alcoholic steatohepatitis (NASH) relative to those observed in the absence of the compound.

By "increases the intracellular expression" is meant increasing over the baseline, or compared to a control, by 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, or 100%, or 2, 3, 4, 5, 6, 7, 8, 9, 10, 11, 12, 13, 14, 15, 20, 25, 30, 35, 40, 45, 50, or more fold.

Furthermore, as already mentioned in the definitions above, downregulator or inihitor compounds are thus those molecules that decrease p63 functional activity or alter its intracellular distribution.

By "decreases the intracellular expression" is meant decreasing below the baseline, or compared to a control, by 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, or 100%, or 2, 3, 4, 5, 6, 7, 8, 9, 10, 11, 12, 13, 14, 15, 20, 25, 30, 35, 40, 45, 50, or more fold.

Activator compounds can be identify by a method for screening a compound for the ability to activate p53, comprising contacting a cell with a compound suspected to activate p53; assaying the contents of the cells to determine the amount and/or biological activity of p53; and comparing the determined amount and/or biological activity of p53 to a predetermined level, wherein a change of said amount and/or biological activity of p53 is indicative for a compound that activates p53. Preferred is a method according to the invention, wherein the cell is a hepatocyte. Further preferred is a method according to the invention, wherein the amount of p53 is determined. In one preferred embodiment, screening is done by quantitative real-time RT-PCR using specific primers for each isoform.

In certain embodiments, such activator compound is a peptide/protein which comprises the sequence of protein p53 or a variant of this sequence which is at least 70%, 75%, 80%, 85%, 90%, 93%, 95%, 96%, 97%, 98% or 99% identical to it.

In another embodiment, the agent is a down-regulator or inhibitor.

Peptides/proteins of the invention may be modified for example by the addition of histidine residues to assist their identification or purification or by the addition of a signal sequence to promote their secretion from a cell where the polypeptide does not naturally contain such a sequence.

A peptide/protein of the invention above may be labelled with a revealing label. The revealing label may be any suitable label, which allows the peptide to be detected.

The peptides/proteins of the invention may be introduced into a cell, such a hepatocyte, by in situ expression of the peptide from a recombinant expression vector. The expression vector optionally carries an inducible promoter to control the expression of the polypeptide.

A peptide/protein of the invention can be produced in large scale following purification by high pressure liquid chromatography (HPLC) or other techniques after recombinant expression.

In another preferred embodiment of the first aspect of the invention, said agent is an activator compound selected from the group consisting of doxorubicin analogues. In the context of the present invention, a doxorubicin analogue includes those compounds in which the aglycone moiety is linked to a different carbohydrate as well as those obtained by derivatization of the biosynthetic glycosides. Doxorubicin analogues can be alternatively, and often preferably, obtain from the corresponding daunorubicin analogues by the introduction of the alcohol function at C-14. Preferably, a doxorubicin analogue useful for the present invention is daunorubicin.

In other embodiment of the invention, the activator compound is a DNA polynucleotide having a sequence selected from:

a. a DNA sequence encoding protein p53 or the complementary sequence thereto;
b. a sequence which selectively hybridizes under stringent conditions to sequence (a);
c. a DNA sequence which is at least 70%, 75%, 80%, 85%, 90%, 93%, 95%, 96%, 97%, 98% or 99% identical to sequence (a) or (b); or
d. a DNA sequence encoding a peptide sequence comprising an amino acid sequence which is at least 70%, 75%, 80%, 85%, 90%, 93%, 95%, 96%, 97%, 98% or 99% identical to the sequence of protein p53.

In another preferred embodiment of the first aspect of the invention, the agent capable of up-regulating the expression of p53 or the activator compound is a DNA polynucleotide having a sequence selected from:

a. a DNA sequence encoding protein p53 or the complementary sequence thereto; or
b. a sequence which selectively hybridizes under stringent conditions to sequence (a).

In yet another preferred embodiment of the first aspect of the invention, the agent capable of down-regulating or inhibiting the expression of p63 is a DNA polynucleotide having a sequence selected from:

a. a DNA sequence encoding a p63$\alpha$ negative dominant or the complementary sequence thereto;
b. a sequence which selectively hybridizes under stringent conditions to sequence (a); or
c. a DNA sequence which is at least 70%, 75%, 80%, 85%, 90%, 93%, 95%, 96%, 97%, 98% or 99% identical to sequence (a) or (b).

In still another preferred embodiment of the first aspect of the invention, the agent capable of down-regulating or inhibiting the expression of p63 is a DNA polynucleotide having a sequence selected from:

a. DNA sequence encoding a p63 negative dominant or the complementary sequence thereto; or
b. a sequence which selectively hybridizes under stringent conditions to sequence (a).

In another aspect of the invention, the activator compound or down-regulator or inhibitor is a mRNA polynucleotide having any of the above mentioned sequences.

The polynucleotides of the invention may include within them synthetic or modified nucleotides. A number of different types of modifications to polynucleotides are known in the art. These include methylphosphate and phosphorothioate backbones, addition of acridine or polylysine chains at the 3'and/or 5'ends of the molecule. For the purposes of the present invention, it is to be understood that the polynucleotides described herein may be modified by any method available in the art.

Polynucleotides such as a DNA polynucleotide according to the invention may be produced recombinantly, synthetically or by any means available to those skilled in the art. They may also be cloned by standard techniques. The polynucleotides are typically provided in isolated and/or purified form.

In a further preferred aspect of the invention, the polynucleotides of the invention, such as those discussed above, can be transported into the hepatocytes, without degradation, by plasmid or viral vectors that include a promoter yielding expression of the nucleic acid in the cells into which it is delivered.

Thus, in a further embodiment of the invention the activators compounds or the down-regulators or inhibitors of the invention can comprise any of the disclosed above polynucleotides of the invention or a plasmid or vector capable of transporting or delivering said polynucleotides, preferably a viral vector.

Viral vectors are, for example, Adenovirus, Adeno-associated virus, Herpes virus, Vaccinia virus, Polio virus, AIDS virus, neuronal trophic virus, Sindbis and other RNA viruses, including these viruses with the HIV backbone. Also preferred are any viral families which share the properties of these viruses which make them suitable for use as vectors.

The activators compounds can comprise, in addition to the disclosed pharmaceutical drugs such as doxorubicin or doxorubicin analogues, polynucleotides of the invention, plasmid or vectors or the peptides of the invention, for example, lipids such as liposomes, such as cationic liposomes (e.g., DOTMA, DOPE, DC-cholesterol) or anionic liposomes.

Liposomes can further comprise proteins to facilitate targeting a particular cell, if desired. Administration of a composition comprising a compound and a cationic liposome can be administered to the blood afferent to a target organ. Furthermore, the activator can be administered as a component of a microcapsule that can be targeted to specific cell types, such as cardiomyocytes, or where the diffusion of the compound or delivery of the compound from the microcapsule is designed for a specific rate or dosage.

The DNA polynucleotides of the invention, such as the ones disclosed above, that are delivered to hepatocytes can be integrated into the host cell genome, typically through integration sequences. These sequences are often viral related sequences, particularly when viral based systems are used. These viral integration systems can also be incorporated into nucleic acids which are to be delivered using a non-nucleic acid based system of deliver, such as a liposome, so that the nucleic acid contained in the delivery system can become integrated into the host genome.

Other general techniques for integration into the host genome include, for example, systems designed to promote homologous recombination with the host genome. These systems typically rely on sequence flanking the nucleic acid to be expressed that has enough homology with a target sequence within the host cell genome that recombination between the vector nucleic acid and the target nucleic acid takes place, causing the delivered nucleic acid to be integrated into the host genome. These systems and the methods necessary to promote homologous recombination are known to those of skill in the art.

The activator compounds or the down-regulators or inhibitors herein disclosed can be administered in a pharmaceutically acceptable carrier and can be delivered to the subject's hepatocytes in vivo and/or ex vivo by a variety of mechanisms well known in the art as commented above.

If *ex vivo* methods are employed, cells or tissues can be removed and maintained outside the body according to standard protocols well known in the art. The activator compounds can be introduced into the cells, preferably hepatocytes, via any gene transfer mechanism, such as, for example, calcium phosphate mediated gene delivery, electroporation, microinjection or proteoliposomes. The transduced cells can then be infused (e.g., in a pharmaceutically acceptable carrier) or homotopically transplanted back into the subject per standard methods for the cell or tissue type. Standard methods are known for transplantation or infusion of various cells into a subject.

The activators compounds or the down-regulators or inhibitors of the present invention can be used in conjunction with another treatment method.

A second aspect of the invention refers to a combination therapy comprising at least two agents, wherein at least one agent is characterized by being capable of up-regulating the expression of p53 and the other agent is characterized by being capable of down-regulating or inhibiting the expression of p63 in the hepatocyte cells of a human subject suffering from non-alcoholic fatty acid disease (NAFLD) or non-alcoholic steatohepatitis (NASH) relative to that observed in the absence of the agent, for use in the concomitant administration (understood as including both simultaneous and sequential administration of the two agents) for the treatment of non-alcoholic fatty acid disease (NAFLD) and/or non-alcoholic steatohepatitis (NASH). Preferably, this aspect of the invention refers to a specific combination therapy comprising doxorubicin or doxorubicin analogues and a further active ingredient, wherein this further active ingredient is preferably quercetin.

A third aspect of the invention refers to a composition comprising an agent or the combination therapy as defined in any of the precedent aspects, wherein said composition is optionally a pharmaceutical composition optionally comprising a pharmaceutically acceptable vehicle and/or pharmaceutically acceptable excipients, for use in the treatment of non-alcoholic fatty acid disease (NAFLD) and/or non-alcoholic steatohepatitis (NASH). In a preferred embodiment, said composition is a plasmid or vector capable of transporting or delivering the polynucleotides as defined herein, preferably a viral vector as defined above. More preferably, said composition is a vaccine composition comprising said plasmids or vectors, preferably viral vectors, capable of transporting or delivering the polynucleotides aforementioned.

The present treatment methods also include a method to increase the efficacy of other agents given for the same disease, comprising administering to a subject in need thereof an effective amount of an activator compound; and, optionally, a pharmaceutically acceptable carrier, thereby increasing the efficacy of the other agent or agents.

In any case, the compositions comprising the activator compound or the down-regulator or inhibitor can be administered *in vivo* in a pharmaceutically acceptable carrier. By "pharmaceutically acceptable" is meant a material that is not biologically or otherwise undesirable, i.e., the material may be administered to a subject, along with the nucleic acid or vector, without causing any undesirable biological effects or interacting in a deleterious manner with any of the other components of the pharmaceutical composition in which it is contained. The carrier would naturally be selected to minimize any degradation of the active ingredient and to minimize any adverse side effects in the subject, as would be well known to one of skill in the art.

As already stated for doxorubicin, effective dosages and schedules for administering the compositions comprising the activator compound disclosed herein may be determined empirically, and making such determinations is within the skill in the art. The dosage ranges for the administration of the compositions are those large enough to produce the desired effect in the disorder. The dosage should not be so large as to cause adverse side effects, such as unwanted cross-reactions, anaphylactic reactions, and the like. Generally, the dosage will vary with the age, condition, sex and extent of the disease in the patient, route of administration, or whether other drugs are included in the regimen, and can be determined by one of skill in the art. The dosage can be adjusted by the individual physician in the event of any contraindications. Dosage can vary, and can be administered in one or more dose administrations daily, for one or several days. Guidance can be found in the literature for appropriate dosages for given classes of pharmaceutical products.

The following examples merely serve to illustrate the present invention.

## Examples 1. Materials and methods and results of the experiments that resulted in figures 1 to 5.

- **Materials and methods**

### Ethics statement

All experimental procedures with animals were performed in compliance with the European Communities directive of 24 November 1986 (86/609/ECC) and Spanish legislation (BOE 252/ 34367-91, 2005) regulating animal research. Research procedures included in the present study were approved by the Research and Bioethics Committee of University of Santiago de Compostela.

### Animals and housing

Adult male Swiss or C57/B6 mice (Animal house, University of Santiago de Compostela) were used. All animals

were experimentally naive, and they were individually housed in controlled room conditions (temperature: 22±2°C; humidity: 40±5%; 12-h light-dark cycle, lights on at 8:00 a.m.) with free access to food and tap water.

**Sample collection**

Animals were killed by decapitation 2 hours after the last dose of treatment in a separate room from the other experimental animals. Blood samples were briefly collected and centrifuged (1000 g for 10 minutes at 4°C), and all plasma samples were frozen at -80°C for biochemical and hormonal analysis. Livers were dissected out. Part of each liver was fixated with 4% paraformaldehyde in 0.1 M phosphate buffered saline (PBS) by immersion until immunohistochemical analysis. The remaining of each sample was briefly frozen at -80°C until RT-qPCR and Western blot analyses.

**Measurement of metabolites**

Serum activities of ALT and AST were measured using the ALT and AST Reagent Kit (Biosystems Reagents) with a Benchmark Plus Microplate Spectrophotometer.

**Hematoxilin/eosin staining**

Liver samples were fixed in 10% buffered formalin for 24 hr, and then dehydrated and embedded in paraffin by a standard procedure. Sections of 3 μm were cut with a microtome and stained using a standard Hematoxilin/Eosin Alcoholic procedure according to the manufacturer's instructions (BioOptica, Milan, Italy). They were then mounted with permanent (non-alcohol, non-xylene based) mounting media, and evaluated and photographed using a BX51 microscope equipped with a DP70 digital camera (Olympus, Tokyo, Japan).

**Oil Red O staining**

Frozen sections of the livers (8 μm) were cut and stained in filtered Oil Red O for 10 minutes. Sections were washed in distilled water, counterstained with Mayers's haematoxylin for 3 minutes and mounted in aqueous mountant (glycerin jelly). Oil Red O quantification was performed using the Image J software (Fiji-win64 version) to determine the amount of staining (using the parameter "IntDen" (the product of Area and Mean Gray Value) related to the cell number (nucleus, DAPI-stained).

**TG content in liver**

Livers (aprox 200 mg) were homogenized for 2 min in ice-cold chloroform-methanol (2:1, vol/vol). TG were extracted during 5-h shaking at room temperature. For phase separation, $H_2SO_4$ was added, samples were centrifuged, and the organic bottom layer was collected. The organic solvent was dried using a Speed Vac and re-dissolved in chloroform. TG (Randox Laboratories LTD, UK) content of each sample was measured in duplicate after evaporation of the organic solvent using an enzymatic method.

***Western blot analysis***

Total protein lysates from liver (20 μg), were subjected to SDS-PAGE, electrotransferred onto a polyvinylidene difluoride membrane and probed with the indicated antibodies: Fatty Acid Synthase (FAS) (H-300) (sc-20140), Lipoproteina lipase (LPL Antibody H-53) (sc-32885), JNK 1/3 (C-17) (sc-474), (Snta Cruz Biotechnology, Santa Cruz, CA); Phospho-SAP/JNK (Thr183/Tyr185) (81E11) Rabbit mAb (#4668), Cleaved Caspase 3 (Asp175) (#9664), (Cell Signaling, Danvers, MA); Monoclonal anti-GAPDH mouse (CB1001) (Upstate, Lake Placid, NY). For protein detection we used horseradish peroxidase-conjugated secondary antibodies and chemiluminescence (Amersham Biosciences, Little Chalfont, UK). Protein levels were normalized to GAPDH for each sample.

***Quantitative reverse transcriptase PCR (qRT-PCR) analysis***

RNA was extracted using Trizol® reagent (Invitrogen) according to the manufacturer's instructions. 2 μg of total RNA were used for each RT reaction, and cDNA synthesis was performed using the SuperScript™ First-Strand Synthesis System (Invitrogen) and random primers. Negative control reactions, containing all reagents except the sample were used to ensure specificity of the PCR amplification. For analysis of gene expression we performed real-time reverse-transcription polymerase chain reaction (RT-PCR) assays using a fluorescent temperature cycler (TaqMan®; Applied Biosystems, Foster City, CA, USA) following the manufacturer's instructions. 500 ng of total RNA were used for each

RT reaction. The PCR cycling conditions included an initial denaturation at 50°C for 10 min followed by 40 cycles at 95°C for 15 sec and 60°C for 1 min. For analysis of the data, the input value of gene expression was standardized to the HPRT value for the sample group and expressed as a comparison with the average value for the control group. All samples were run in duplicate and the average values were calculated.

**Data Analysis and Statistics**

Values are plotted as the mean ± SEM for each genotype. Statistical analysis was performed using a Student's t -test. A P value less than 0.05 was considered statistically significant.

- **Results**

In order to demonstrate the usefulness of this drug, the authors of the present invention have conducted a series of experiments in which 0.3, 0.6, 1.25, 2.5 and 5 mg/kg of doxorubicin were administered intra-peritoneally to mice of the Swiss strain fed with a standardize diet. In these experiments it can be observed how the latter mentioned dosages provoked a significant loss of weight as shown in figure 1A in a dose dependent manner, wherein only dosages greater or equal to 0.6 mg/kg were capable of reducing the body weight of the mice significantly (see Figures 1A and 1B).

Based on these results, the authors of the present invention conducted a further series of experiments using doxorubicin in an animal model of hepatic steatosis, in particular in an animal model of HFD-induced obese mice. This specific animal model suffers from hepatic steatosis and accurately reproduces human obesity.

In these further series of experiments, as already detailed above, mice were fed with a HFD (45% total fat content) during a period of 12 weeks, and after said period of time, mice were treated intra-peritoneally with 0.15, 0.3, 0.6 and 1.25 mg/kg of doxorubicin during a further period of 2 months. During this two month period, doxorubicin was administered twice per week using each of the different dosages above mentioned.

After said period of two months, we observed a significant loss of body weight at all studied dosages as clearly reflected in Fig. 2A. Moreover, these experiments demonstrate that a dose of 0.6 mg/kg body weight is remarkably reduced without altering the normal food intake of the mice as reflected in Fig. 2B. This latter mentioned result suggests that the use of doxorubicin (at this dosage) does not result in any significant adverse effect, more so when the reduction in body weight has been shown to correspond to a reduce quantity of fat without altering the muscle mass as shown in figure 2C.

In addition, we determined whether the intra-peritoneal administration of dosages of 0,6 mg/kg provoked cardiotoxicity in the animal model. For this purpose, we determined the levels of troponine, a marker of cardiovascular damage, after the administration of the latter mentioned dosage. As shown in figure 2D, doxorubicin at a fixed dose of 0,6 mg/kg, fails to significantly increase the levels of troponine. This result clearly suggests the absence of cardiotoxic effects of doxorubicin at this dose.

Once we have determined that dosages of 0,6 mg/kg fail to result in significant adverse effects in the studied animal model, we conducted a further series of studies specifically directed to the liver. First of all, we conducted a histological study and observed that obese mice treated with doxorubicin at a dosage of 0,6 mg/kg during a period of time of two months have reduced hepatic damage as shown in figure 3A. Additionally, the level of triglycerides in the liver of these mice is also reduced in comparison to animals treated with a control vehicle while the level of non-esterified fatty acids in their liver is increased (see figure 3B). This suggests that doxorubicin increases the oxidation of fatty acids in the liver.

At a molecular level, we found that treatment with doxorubicin (at a dose of 0,6 mg/kg) during a two month period, reduces the expression of inflammatory markers such as TNF-a, arginase, IL-6, NOS2 and F480 (see figure 3C). This specific type of treatment also reduced the protein levels of genes implicated in ER stress (please note that this type of stress is proportional to the severity of the steatosis) such as pIRE, XBP1, CHOP y pJNK (see figure 3D)

Furthermore, the authors of the present invention in order to corroborate the above mentioned results, conducted a still further series of experiments by using mice of the C57/B6 strain fed with a HFD (60% total fat content) during a period of 12 weeks. Subsequently, mice were administered a dose of 0,6 mg/kg intra-peritoneally twice per week during a further period of two months. The results shown in figures 4A and C indicate that mice reduced their body weight significantly and that hepatic tryglicerides were also significantly reduced.

Moreover, the authors conducted a series of experiments in which a vehicle, quercetin 15 mg/kg, quercetin 15mg/Kg + ADR 20mg/Kg and quercetin 15mg/Kg + ADR 10mg/Kg were administered orally twice per week during a period of 30 days to mice of the C57/B6 strain fed with a HFD (60% total fat content) during a period of two months. In these experiments, as shown in figure 5, it can be observed how the latter mentioned dosages (quercetin 15mg/Kg + ADR 20mg/Kg and quercetin 15mg/Kg + ADR 10mg/Kg) provoked a reduction in the body weight and a reduction in hepatic tryglicerides, particularly in the case of quercetin 15mg/Kg + ADR 20mg/Kg.

**Example 2. Materials and methods and results of the experiments that resulted in figures 6 to 11.**

- **Materials and methods**

*1. Animals*

Animal protocols were approved by the Committee at the University of Santiago de Compostela. p53 null mice showed the expected shorter lifespan and tumour spectrum.

*2. Histological procedures*

Hematoxilin/eosin staining and oil red were performed.

*3. TG content in liver*

The extraction procedure for tissue lipids was adapted from methods described previously.

*4. Quantitative reverse transcriptase PCR (qRT-PCR) analysis*

RNA was extracted using Trizol® reagent (Invitrogen) according to the manufacturer's instructions.

*5. Cell culture and adenoviral transduction*

HepG2 cells were infected with adenoviruses expressing GFP alone or adenoviruses encoding a p53 negative dominant and treated with etoposide.

*6. Tail vein injections for in vivo adenoviral gene transfer*

Adenoviral vectors targeting p53 or p63 were injected via tail vein injection.

*7. Data Analysis and Statistics*

Values are plotted as the mean ± SEM for each genotype. Statistical analysis was performed using a Student's t -test. A P value less than 0.05 was considered statistically significant.

- **Results**

**1. Male p53 null mice fed a high fat diet are resistant to diet-induced weight gain and insulin resistance**

Age-matched male WT and p53 null mice were maintained on standard diet from 4 weeks of age for 8 weeks to assess their metabolic phenotypes. No differences were found in body weight, food intake or body composition (Figures 7A-C). Another group of age-matched male WT and p53 null mice was maintained on HFD from 4 weeks of age (45% kcal fat, 4.73 kcal/g) for 11 weeks. p53 null mice on a HFD gained significantly less body weight than WT mice (Figure 6A). Changes in body weight could not be explained to daily reductions in total food intake (Figure 6B). Body composition revealed that p53 null mice accrued less fat mass compared to WT mice after 11 weeks on a HFD with no changes in non-fat mass (Figure 6C). Male p53 null mice fed a chow diet did not show any alteration in glucose tolerance (Figure 6D) or insulin sensitivity (Figure 6E). Male p53 null mice fed a chow diet did not show changes in glucose tolerance (Figure 1D) but when they were fed a HFD had increased insulin sensitivity (Figure 6E). Female p53 null mice maintained on standard diet did not show any alteration in body weight, food intake or body composition. When females were fed a HFD they had a significant decrease in fat mass compared to female WT mice but no differences in body weight, glucose tolerance nor insulin sensitivity.

**2. p53 null mice fed a chow diet or high fat diet have increased hepatic steatosis and steatohepatitis**

Male p53 null mice exhibited more lipid droplets in their hepatocytes, compared with those observed in their WT littermates independent of the type of diet received (Figure 7A). Consistently, p53 null mice had more triglicerydes (TG) in the liver and increased serum aspartate aminotransferase (AST) and alanine transaminase (ALT) levels than WT mice (Figure 7B). We found that mRNA expression of PPARγ, a transcription factor that is responsible for the lipid

accumulation in hepatic steatosis, and SCARB1, which controls high-density lipoprotein re-uptake were up-regulated in the liver of p53 null mice compared to WT mice (Figures 7C). Protein levels of FAS and LPL, as well as hepatic LPL activity, were increased in the liver of p53 deficient mice when compared to their WT littermates (Figure 7C-D).

p53 deficient mice exhibited increased hepatic protein levels of the cleaved caspase 3 and cleaved caspase 7 (Figure 7D). Since ER stress has been implicated in the pathogenesis of NAFLD and NASH, we next assessed the protein levels of several key factors mediating ER stress. We found that in the liver of p53 deficient mice, the hepatic levels of pIRE/IRE, XBP1, pPERK, and peIF2$\alpha$/IF2$\alpha$, members of the unfolded protein response to ER stress, were significantly increased in comparison to WT controls (Figure 7D).

In contrast to the elevated TG levels in the liver, serum TG levels were lower in p53-deficient mice compared to WT controls fed a chow diet, whereas NEFAs, cholesterol and glucose levels remained unchanged between both genotypes. P53 null mice fed a high fat diet showed lower TG and NEFAs serum levels with unchanged cholesterol and glucose..

**3. Hepatic inactivation of p53 causes hepatic steatosis and steatohepatitis**

Using AAV8-mediated Cre-LoxP recombination in p53 floxed mice (p53 flox/flox), we aimed to examine the role of the specific down-regulation of hepatic p53 on liver condition. Protein was isolated from the liver of AAV-infected mice and subsequently tested by western blot to detect the excised p53. p53 protein was excised by the Cre-recombinase 1 month after the tail vein injection of Cre-AAV8 (Figure 8A). The inactivation of p53 in the liver increased the amount of lipid droplets in hepatocytes, compared with those observed in their littermates controls (Figure 8B). Hepatic TG levels and serum aspartate aminotransferase (AST) levels were also elevated when p53 was inactivated in the liver (Figure 8C). In agreement with an impaired hepatic status, protein levels of FAS, LPL and pJNK/JNK, several markers of ERstress (pIRE/IRE, XBP1, pPERK, and peIF2$\alpha$/IF2$\alpha$) and cleaved caspases 3 and 7 were increased after the inactivation of hepatic p53 when compared to their control littermates (Figure 8D). In spite of the impaired hepatic condition, glucose tolerance and insulin sensitivity remained unaltered in these mice (Figure 8E-F).

**4. The inactivation of p53 impairs the response of HepG2 cells to etoposide**

HepG2 cells were infected with adenoviruses expressing GFP alone or adenoviruses encoding a p53 negative dominant. Infection efficiency was assessed by decreased expression of phosphor-p53 (pp53) (Figure 9A). In order to investigate the response of control cells and cells with silenced p53, we challenged them to etoposide, a well established compound that induces stress. As expected, etoposide increased lipid deposition in HepG2 cells in a dose-dependent manner, and similarly to the results observed in mice models, p53 dominant negative-infected cells showed an increased amount of lipid droplets in comparison to control cells when treated with etoposide (Figure 9B). The impaired response to etoposide of cells with silenced p53 was consistent with the higher levels of ER stress markers such as peIF2$\alpha$ and XBP1 compared to control cells (Figure 9C).

**5. Hepatic activation of p53 ameliorates hepatic steatosis and steatohepatitis in wild type and p53 KO mice fed a HFD**

Having shown that both complete and liver-specific lack of p53 caused hepatic steatosis and steatohepatitis, we next tested if the specific recovery of p53 in the liver was sufficient to reverse the hepatic effects of p53 deficiency. *In vivo* adenoviral gene transfer to activate p53 in the liver was accomplished by tail vein injection of adenoviruses encoding either GFP or p53. GFP was specifically detected in the liver (but not in other tissues such as BAT) of WT and p53 null mice (Figure 10A) and hepatic levels of p53 were significantly elevated following the injection of Ad-p53 for 1 week compared with mice injected with Ad-GFP (Figure 10B). One week after the injection of adenoviral particles activating p53, both WT and p53 null mice fed a HFD exhibited less lipid droplets in their hepatocytes, compared with those observed in mice injected with scramble adenoviruses (Figure 10C). Consistent with these data, we also found decreased total hepatic TG content in Ad-p53 mice in comparison to Ad-GFP-treated mice (Figure 10D) and a tendency to lower levels of serum AST that were not statistically significant (Figure 10C). The ameliorated hepatic steatosis and steatohepatitis in WT and p53 null mice following Ad-p53 injection was caused by the inhibition of hepatic FAS, LPL, pJNK/JNK and ER stress as demonstrated by the down-regulation of ER stress markers such as pPERK, pEIF2$\alpha$, pIRE, and XBP1 and by the decreased levels of cleaved caspase 3 (Figures 10E).

**6. Hepatic down-regulation of p63 ameliorates hepatic steatosis in mice with hepatic inactivation of p53**

Downstream target genes of p53 has been previously linked to the regulation of lipid metabolism include bax or p66shc. However, we failed to detect significant changes in the expression of those genes. We assessed p63 levels

in the liver of p53 genetically engineered mice models. We found that hepatic p63 levels were increased in p53 null mice (Figure 11A) and in mice with specific down-regulation of hepatic p53 (Figure 11B). In agreement with those results, hepatic p63 levels were decreased when p53 expression was recovered in p53 null mice (Figure 11C).

Given the negative correlation between p53 and p63, we next sought to investigate if the down-regulation of hepatic p63 could reverse the hepatic damage of mice lacking p53 in the liver. To this aim, we used adeno-associated virus (AAV)-mediated Cre-LoxP recombination in p53 floxed mice (p53 flox/flox) together with lentivirus expressing GFP alone or a lentivirus encoding a p63 shRNA administered in the tail vein to inhibit expression of both p53 and p63 specifically in liver. Infection efficiency of sh-p63 was assessed by decreased p63 protein levels (Figure 6D). One month after the injection of AAV8-Cre and p63 shRNA, mice exhibited less lipid droplets in their hepatocytes compared with those observed in mice injected AAV8-Cre and scramble lentiviruses (Figure 11E). We also found a significant decrease in total hepatic TG content, and serum AST in mice expressing low levels of both p53 and p63 in the liver in comparison to mice lacking hepatic p53 (Figure 11F). The ameliorated hepatic steatosis in mice expressing low levels of both p53 and p63 in the liver was also consistent with the inhibition of hepatic FAS, LPL activity, pJNK and ER stress markers such as pPERK and XBP1, and by the decreased levels of cleaved caspase 3 (Figures 11G)

**7. Dose translation rats to humans**

The amounts of doxorubicine administered to rats have been herein extrapolated to a human dose as described in Reagan-Shaw et al., "Dose translation from animal to human studies revisited", The FASEB Journal, 2007, 22, 659-661, using the equation:

$$\text{Dose in humans (mg/kg)} = \text{Dose in animal (mg/kg)}*(\text{km animal/km human})$$

Using the Km values shown in the table below:

| Species | Weight (kg) | BSA ($m^2$) | $K_m$ factor |
|---|---|---|---|
| Human | | | |
| Adult | 60 | 1.6 | 37 |
| Child | 20 | 0.8 | 25 |
| Baboon | 12 | 0.6 | 20 |
| Dog | 10 | 0.5 | 20 |
| Monkey | 3 | 0.24 | 12 |
| Rabbit | 1.8 | 0.15 | 12 |
| Guinea pig | 0.4 | 0.05 | 8 |
| Rat | 0.15 | 0.025 | 6 |
| Hamster | 0.08 | 0.02 | 5 |
| Mouse | 0.02 | 0.007 | 3 |
| Values based on data from FDA Draft Guidelines (7). To convert dose in mg/kg to dose in mg/$m^2$, multiply by $K_m$ *value.* | | | |

**8. Doxorubicin protects the accumulation of lipids induced by oleic acid in human hepatocytes**

The hepatocyte cell line of human origin HepG2 was treated with bovine serum albumin (BSA, which represents the control group), oleic acid (OA) and 50 nM oleic acid + doxorubicin for 48 h. As expected, oleic acid significantly increases the amount of lipids in the cells. However, in the hepatocytes incubated with oleic acid and doxorubicin a significant decrease in the amount of lipids is observed compared with the cells treated with oleic acid **(Figure 12).**

**Claims**

1. A pharmaceutical composition comprising a compound capable of up-regulating the expression of p53 and/or down-regulating or inhibiting the expression of p63 in the hepatocyte cells of a human being suffering from non-alcoholic fatty liver disease (NAFLD) or non-alcoholic steatohepatitis (NASH), in relation to that observed in the absence of the agent, for use in the prophylactic or therapeutic treatment of non-alcoholic fatty liver disease (NAFLD) and/or non-alcoholic steatohepatitis (NASH).

2. The pharmaceutical composition for use, according to the claim 1, wherein said compound is doxorubicin, a pharmaceutical acceptable salt thereof, or an analogue thereof.

3. The pharmaceutical composition for use according to claim 2, wherein said analogue is daunorubicin or a pharmaceutically acceptable salt thereof.

4. The composition for use according to claim 2, wherein doxorubicin is in pegylated liposomal form or non-pegylated liposomal form.

5. The composition for use according to any of claims 1 to 4, wherein doxorubicin, a pharmaceutical acceptable salt thereof, a liposomal form thereof or an analogue thereof is found in combination with a further active ingredient.

6. The composition for use according to any of claims 1 to 5, wherein said composition is administered in a pharmaceutical form appropriate for the oral, intravenous, intravesical or intra-arterial administration.

7. The composition for use according to claim 6, wherein said pharmaceutical form is appropriate for the oral administration.

8. The composition for use according to claim 6, wherein said pharmaceutical form is appropriate for the intravenous, intravesical or intra-arterial administration.

9. The composition for use according to claim 7, wherein said pharmaceutical oral form comprises doxorubicin and the form is administered to a human being comprising a dosage of the active ingredient doxorubicin of from about 0.8 mg/kg/week (29.6 $mg/m^2$/week) to about 5.0 mg/kg/week (185 $mg/m^2$/week).

10. The composition for use according to claim 7, wherein said pharmaceutical oral form comprises doxorubicin and the form is administered comprising a dosage of the active ingredient doxorubicin of about from 50 mg to about 300 mg.

11. The composition for use of any of claims 7 or 9 to 10, wherein said composition comprises a further pharmaceutical ingredient.

12. The composition for use according to claim 8, wherein said pharmaceutical form comprises doxorubicin and the form is administered to a human being comprising a dosage of the active ingredient doxorubicin of from about 0.024 mg/kg/week (0.9 $mg/m^2$/week) to about 0.20 mg/kg/week (7.5 $mg/m^2$/week).

13. The composition for use according to claim 8, wherein said pharmaceutical form comprises doxorubicin and the form is administered to a human being comprising a dosage of the active ingredient doxorubicin of from about 0.024 mg/kg/week (0.9 $mg/m^2$/week) to about 0.10 mg/kg/week (3.75 $mg/m^2$/week).

14. The composition for use of any of claims 8 or 12 to 13, wherein said composition comprises a further pharmaceutical ingredient.

15. The composition for use according to any of claims 5, 11 or 14, wherein said further pharmaceutical ingredient is the combination of quercetin and doxorubicin.

16. The composition for use as defined in any of claims 1 to 15, wherein such use is in the treatment of morbid obesity in human subjects suffering from non-alcoholic fatty acid disease (NAFLD) and/or non-alcoholic steatohepatitis (NASH).

## Patentansprüche

1. Pharmazeutische Zusammensetzung umfassend eine Verbindung, welche in der Lage ist, die Expression von p53 hochzuregeln und/oder die Expression von p63 herunterzuregeln oder zu hemmen, in den Hepatozyten eines Menschen, welcher unter nichtalkoholischer Fettlebererkrankung (NAFLD) oder nichtalkoholischer Steatohepatitis (NASH) leidet, in Bezug auf diejenige, welche in Abwesenheit des Mittels beobachtet wird, für deren Verwendung bei der vorbeugenden oder therapeutischen Behandlung von nichtalkoholischer Fettlebererkrankung (NAFLD) und/oder nichtalkoholischer Steatohepatitis (NASH).

2. Pharmazeutische Zusammensetzung für deren Verwendung nach Anspruch 1, wobei die genannte Verbindung Doxorubicin, ein pharmazeutisch akzeptables Salz desselben oder ein Analogon desselben ist.

3. Pharmazeutische Zusammensetzung für deren Verwendung nach Anspruch 2, wobei das genannte Analogon Daunorubicin oder ein pharmazeutisch akzeptables Salz desselben ist.

4. Zusammensetzung für deren Verwendung nach Anspruch 2, wobei das Doxorubicin in pegylierter liposomaler Form oder nichtpegylierter liposomaler Form ist.

5. Zusammensetzung für deren Verwendung nach einem der Ansprüche 1 bis 4, wobei das Doxorubicin, ein pharmazeutisch akzeptables Salz desselben, eine liposomale Form desselben oder ein Analogon desselben in Kombination mit einem zusätzlichen Wirkstoff vorliegt.

6. Zusammensetzung für deren Verwendung nach einem der Ansprüche 1 bis 5, wobei die genannte Zusammensetzung in einer Darreichungsform verabreicht wird, welche zur oralen, intravenösen, intravesikalen oder intraarteriellen Verabreichung geeignet ist.

7. Zusammensetzung für deren Verwendung nach Anspruch 6, wobei die genannte Darreichungsform zur oralen Verabreichung geeignet ist.

8. Zusammensetzung für deren Verwendung nach Anspruch 6, wobei die genannte Darreichungsform zur intravenösen, intravesikalen oder intraarteriellen Verabreichung geeignet ist.

9. Zusammensetzung für deren Verwendung nach Anspruch 7, wobei die genannte orale Darreichungsform Doxorubicin umfasst und die Form einem Menschen verabreicht wird, umfassend eine Dosierung des zusätzlichen Wirkstoffes Doxorubicin von etwa 0,8 mg/kg/Woche (29,6 $mg/m^2$/Woche) bis etwa 5,0 mg/kg/Woche (185 $mg/m^2$/Woche).

10. Zusammensetzung für deren Verwendung nach Anspruch 7, wobei die genannte orale Darreichungsform Doxorubicin umfasst und die Form verabreicht wird, umfassend eine Dosierung des zusätzlichen Wirkstoffes Doxorubicin von etwa 50 mg bis etwa 300 mg.

11. Zusammensetzung für deren Verwendung nach einem der Ansprüche 7 oder 9 bis 10, wobei die genannte Zusammensetzung einen zusätzlichen pharmazeutischen Bestandteil umfasst.

12. Zusammensetzung für deren Verwendung nach Anspruch 8, wobei die genannte Darreichungsform Doxorubicin umfasst und die Form einem Menschen verabreicht wird, umfassend eine Dosierung des zusätzlichen Wirkstoffes Doxorubicin von etwa 0,024 mg/kg/Woche (0,9 $mg/m^2$/Woche) bis etwa 0,20 mg/kg/Woche (7,5 $mg/m^2$/Woche).

13. Zusammensetzung für deren Verwendung nach Anspruch 8, wobei die genannte Darreichungsform Doxorubicin umfasst und die Form einem Menschen verabreicht wird, umfassend eine Dosierung des zusätzlichen Wirkstoffes Doxorubicin von etwa 0,024 mg/kg/Woche (0,9 $mg/m^2$/Woche) bis etwa 0,10 mg/kg/Woche (3,75 $mg/m^2$/Woche).

14. Zusammensetzung für deren Verwendung nach einem der Ansprüche 8 oder 12 bis 13, wobei die genannte Zusammensetzung einen zusätzlichen pharmazeutischen Bestandteil umfasst.

15. Zusammensetzung für deren Verwendung nach einem der Ansprüche 5, 11 oder 14, wobei der genannte zusätzliche pharmazeutische Bestandteil die Kombination von Quercetin und Doxorubicin ist.

16. Zusammensetzung für deren Verwendung nach einem der Ansprüche 1 bis 15, wobei solche Verwendung bei der Behandlung von krankhafter Fettleibigkeit in menschlichen Individuen ist, welche unter nichtalkoholischer Fettlebererkrankung (NAFLD) und/oder nichtalkoholischer Steatohepatitis (NASH) leiden.

## Revendications

1. Composition pharmaceutique comprenant un composé apte à réguler à la hausse l'expression de p53 et/ou réguler à la baisse ou inhiber l'expression de p63 dans les cellules hépatocytaires d'un être humain souffrant de la stéatose hépatique non alcoolique (NAFLD) ou stéatohépatite non alcoolique (NASH), par rapport à ce qui est observé dans

l'absence de l'agent, pour son utilisation dans le traitement prophylactique ou thérapeutique de la stéatose hépatique non alcoolique (NAFLD) et/ou la stéatohépatite non alcoolique (NASH).

**2.** Composition pharmaceutique pour son utilisation selon la revendication 1, dans laquelle ledit composé est la doxorubicine, un sel pharmaceutiquement acceptable de celle-ci, ou un analogue de celle-ci.

**3.** Composition pharmaceutique pour son utilisation selon la revendication 2, dans laquelle ledit analogue est de la daunorubicine ou un sel pharmaceutiquement acceptable de celle-ci.

**4.** Composition pharmaceutique pour son utilisation selon la revendication 2, dans laquelle la doxorubicine est sous forme liposomiale pégylée ou sous forme liposomiale non-pégylée.

**5.** Composition pour son utilisation selon l'une quelconque des revendications 1 à 4, dans laquelle la doxorubicine, un sel pharmaceutiquement acceptable de celle-ci, une forme liposomiale de celle-ci ou un analogue de celle-ci se trouve en combinaison avec un principe actif additionnel.

**6.** Composition pour son utilisation selon l'une quelconque des revendications 1 à 5, dans laquelle ladite composition est administrée sous une forme pharmaceutique appropriée pour administration oral, intraveineuse, intra-vésicale ou intra-artérielle.

**7.** Composition pour son utilisation selon la revendication 6, dans laquelle ladite forme pharmaceutique est appropriée pour administration orale.

**8.** Composition pour son utilisation selon la revendication 6, dans laquelle ladite forme pharmaceutique est appropriée pour administration intraveineuse, intra-vésicale ou intra-artérielle.

**9.** Composition pour son utilisation selon la revendication 7, dans laquelle ladite forme pharmaceutique orale comprend de la doxorubicine et la forme est administrée à un être humain comprenant un dosage du principe actif doxorubicine d'environ 0,8 mg/kg/semaine (29,6 $mg/m^2$/semaine) à environ 5,0 mg/kg/semaine (185 $mg/m^2$/semaine).

**10.** Composition pour son utilisation selon la revendication 7, dans laquelle ladite forme pharmaceutique orale comprend de la doxorubicine et la forme est administrée comprenant un dosage du principe actif doxorubicine d'environ 50 mg à environ 300 mg.

**11.** Composition pour son utilisation selon l'une quelconque des revendications 7 ou 9 à 10, dans laquelle ladite composition comprend un principe pharmaceutique additionnel.

**12.** Composition pour son utilisation selon la revendication 8, dans laquelle ladite forme pharmaceutique comprend de la doxorubicine et la forme est administrée à un être humain comprenant un dosage du principe actif doxorubicine d'environ 0,024 mg/kg/semaine (0,9 $mg/m^2$/semaine) à environ 0,20 mg/kg/semaine (7,5 $mg/m^2$/semaine).

**13.** Composition pour son utilisation selon la revendication 8, dans laquelle ladite forme pharmaceutique comprend de la doxorubicine et la forme est administrée à un être humain comprenant un dosage du principe actif doxorubicine d'environ 0,024 mg/kg/semaine (0,9 $mg/m^2$/semaine) à environ 0,10 mg/kg/semaine (3,75 $mg/m^2$/semaine).

**14.** Composition pour son utilisation selon l'une quelconque des revendications 8 ou 12 à 13, dans laquelle ladite composition comprend un principe pharmaceutique additionnel.

**15.** Composition pour son utilisation selon l'une quelconque des revendications 5, 11 ou 14, dans laquelle ledit principe pharmaceutique additionnel est la combinaison de la quercétine et la doxorubicine.

**16.** Composition pour son utilisation selon l'une quelconque des revendications 1 à 15, dans laquelle ladite utilisation est dans le traitement de l'obésité morbide chez des êtres humains souffrant de la stéatose hépatique non alcoolique (NAFLD) et/ou la stéatohépatite non alcoolique (NASH).

**Figure 1**

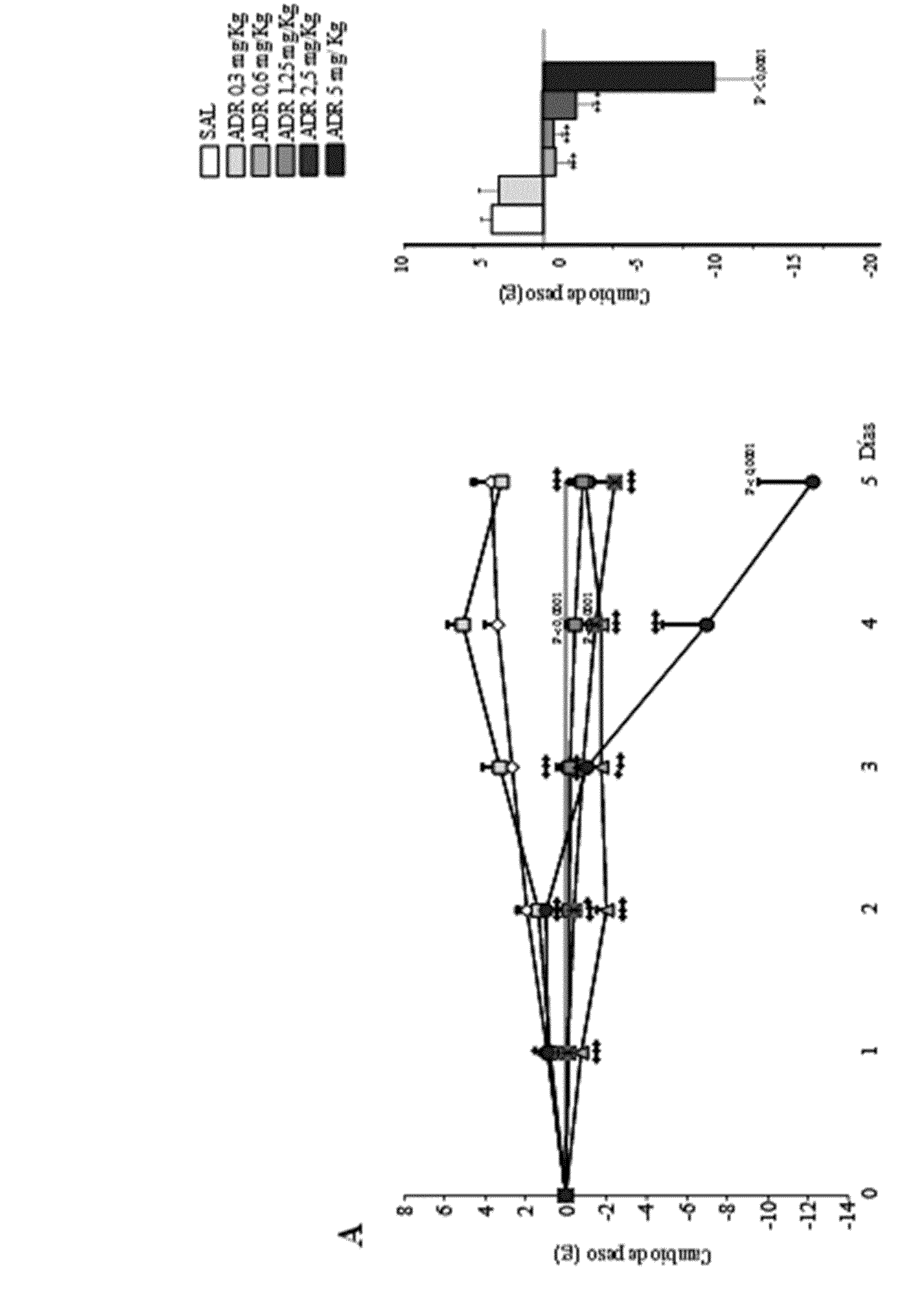
SAL
ADR 0,3 mg/Kg
ADR 0,6 mg/Kg
ADR 1,25 mg/Kg
ADR 2,5 mg/Kg
ADR 5 mg/Kg
Cambio de peso (g)
A
Días

Figure 1 (cont.)

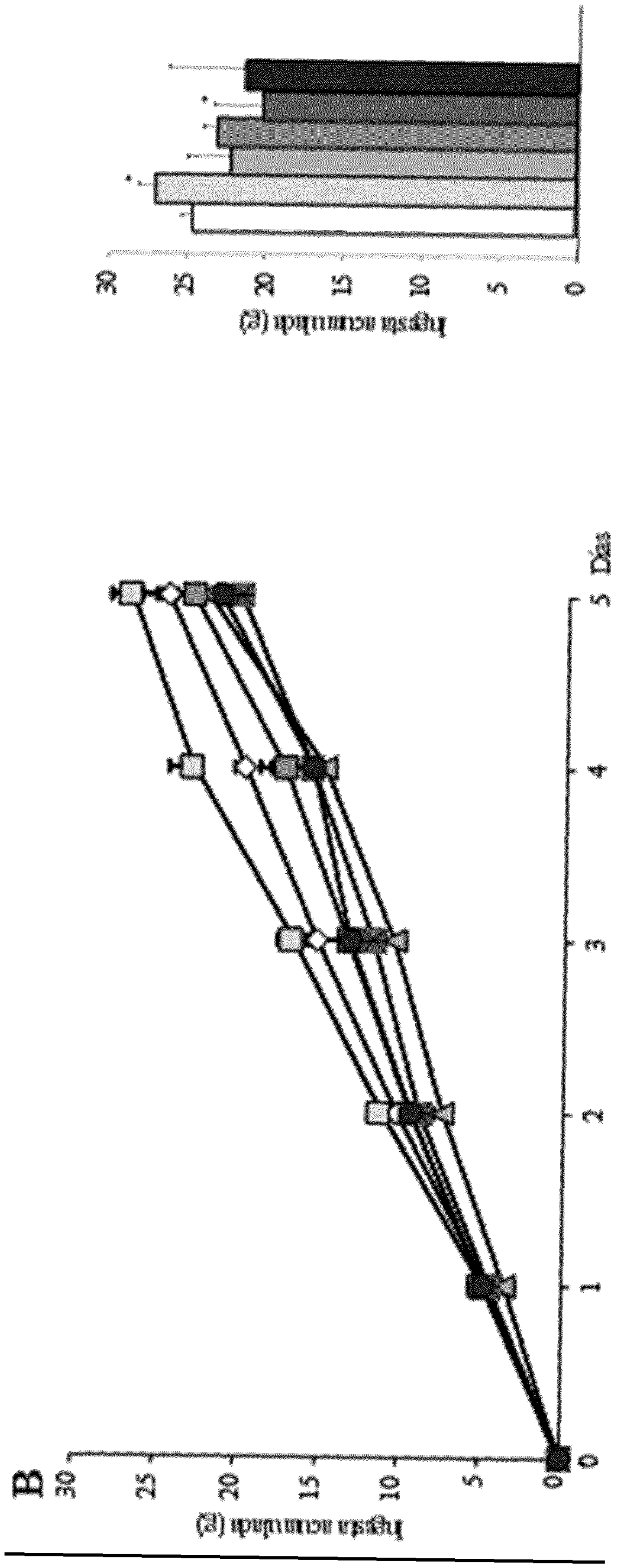

**Figure 2**

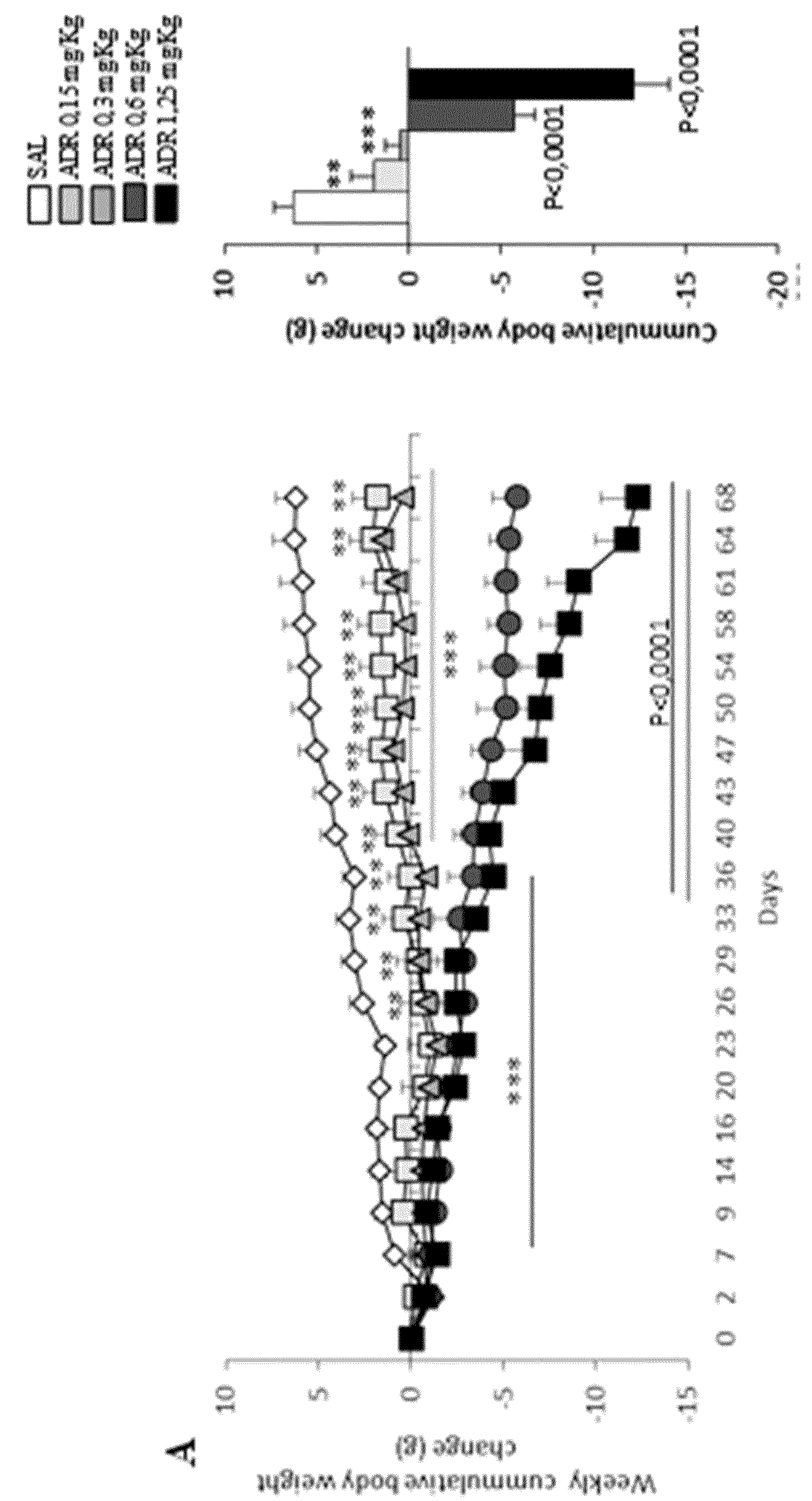
SAL
ADR 0,15 mg/Kg
ADR 0,3 mg/Kg
ADR 0,6 mg/Kg
ADR 1,25 mg/Kg
Cummulative body weight change (g)
P<0,0001
P<0,0001
A
Weekly cummulative body weight change (g)
Days
P<0,0001

**Figure 2 (cont.)**

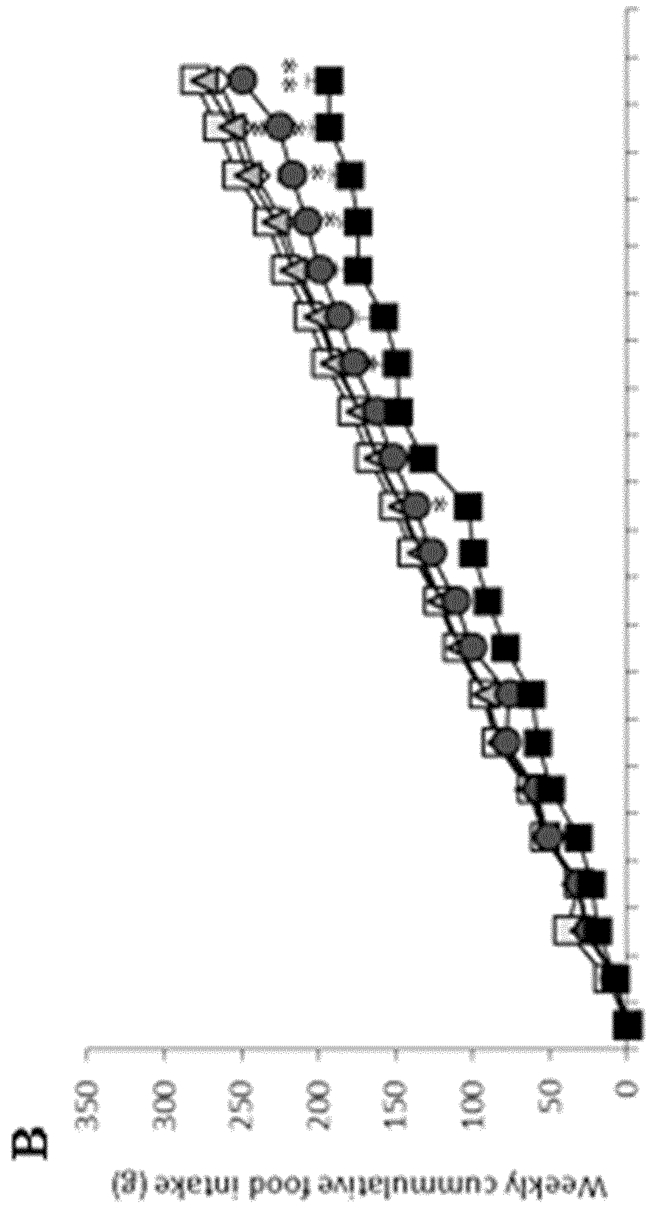


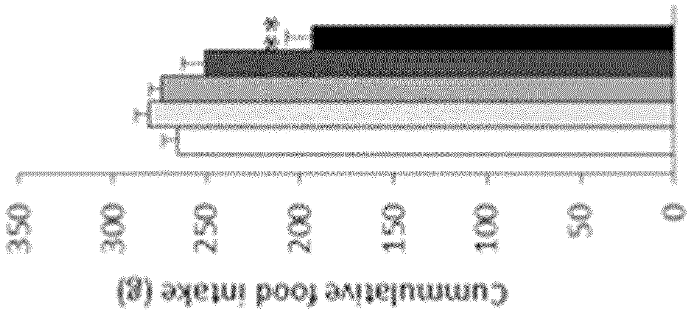

Figure 2 (cont.)

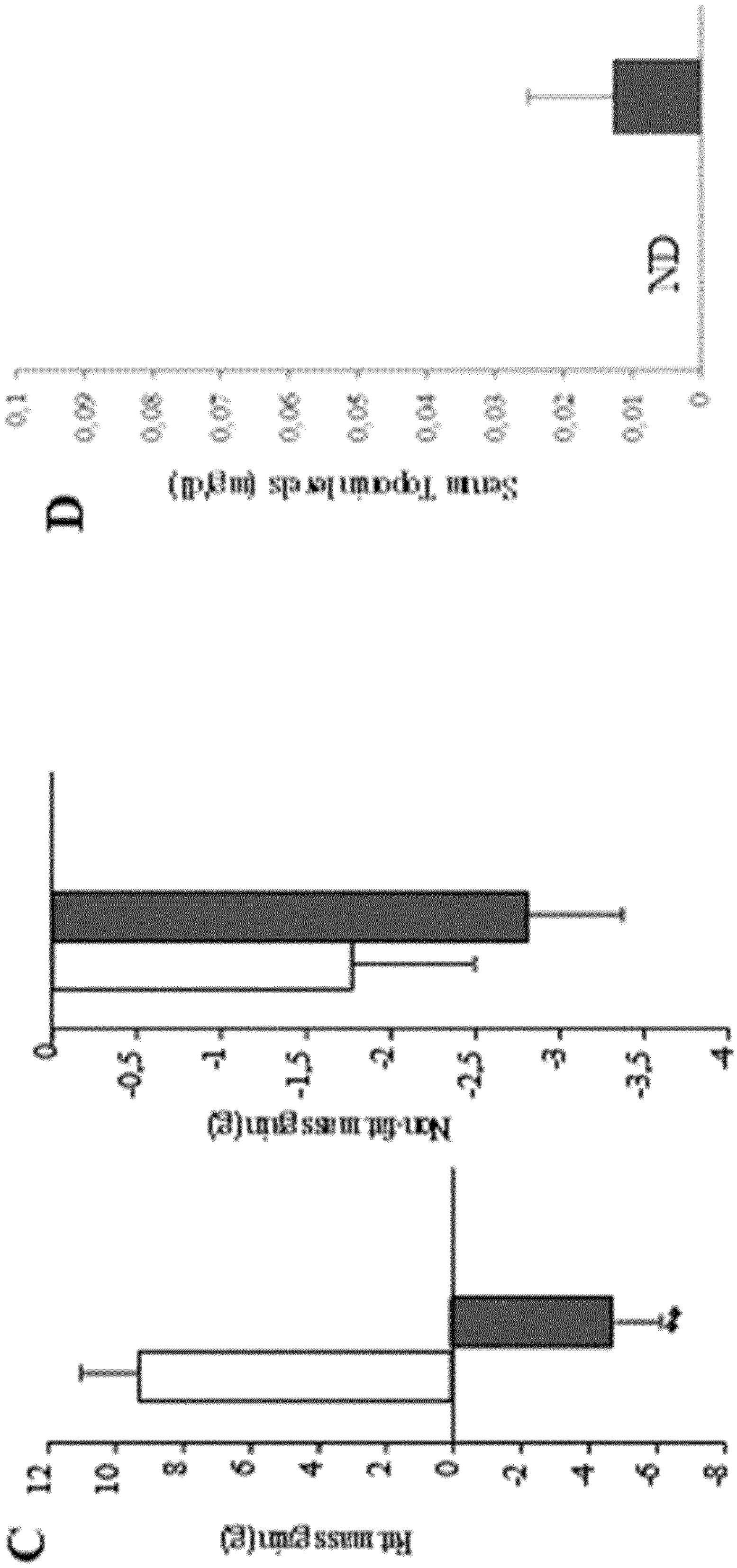

**Figure 3**

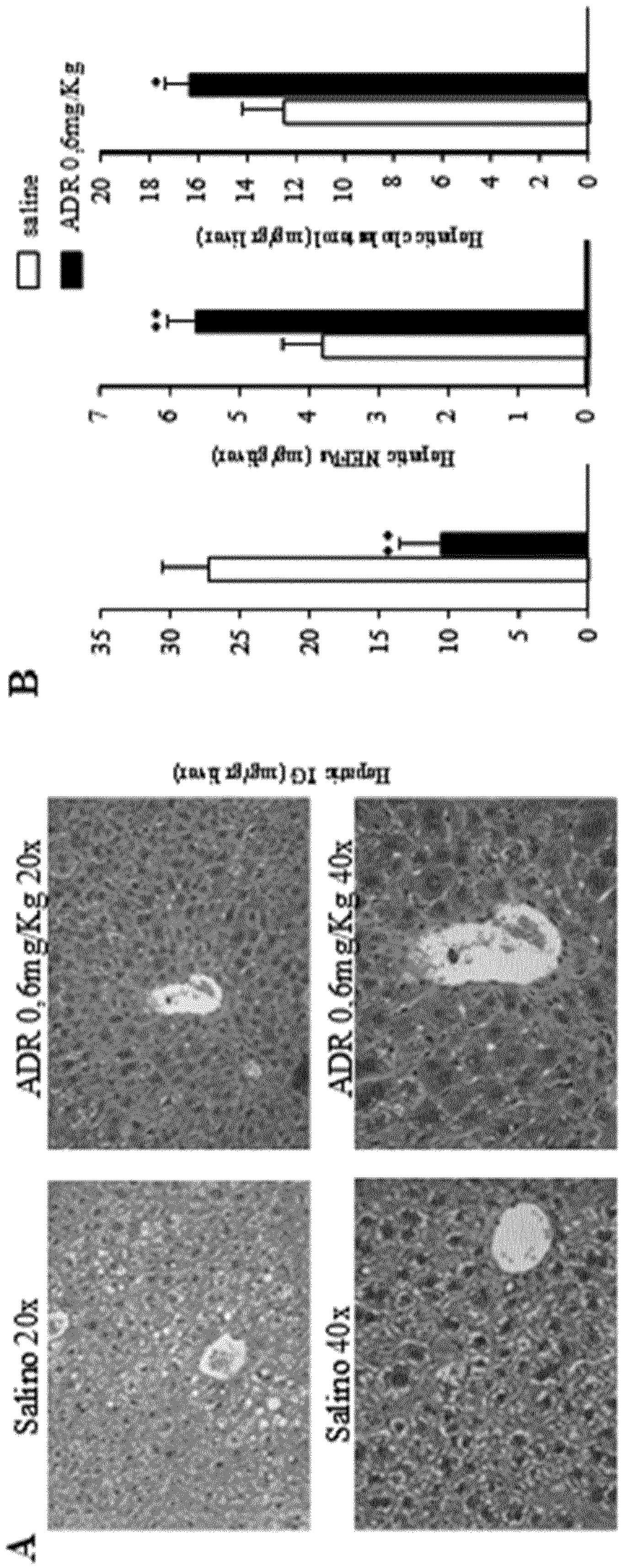
A
Salino 20x
ADR 0,6mg/Kg 20x
Salino 40x
ADR 0.6mg/Kg 40x
B
saline
ADR 0,6mg/Kg

**Figure 3 (cont.)**

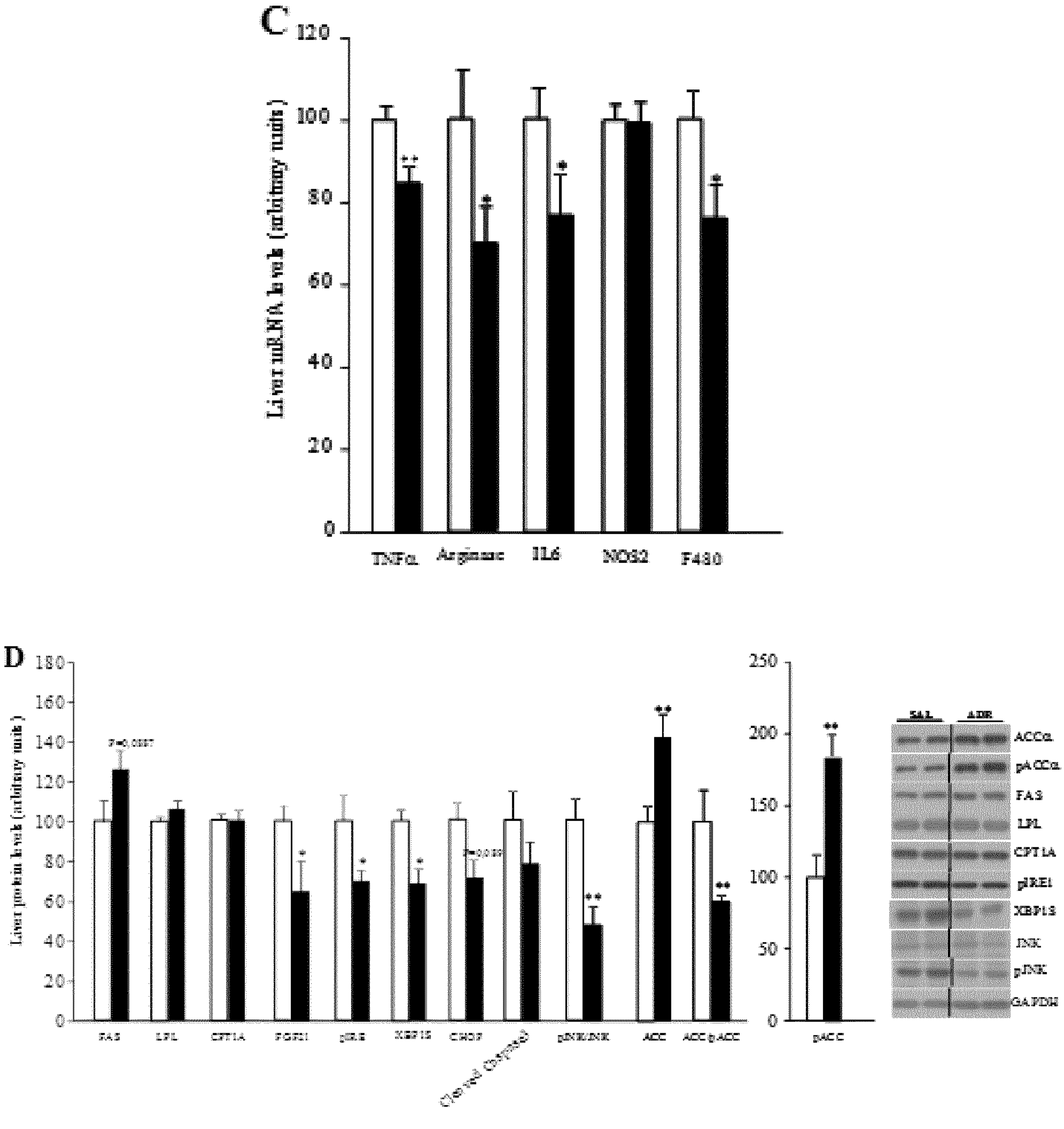

**Figure 4**

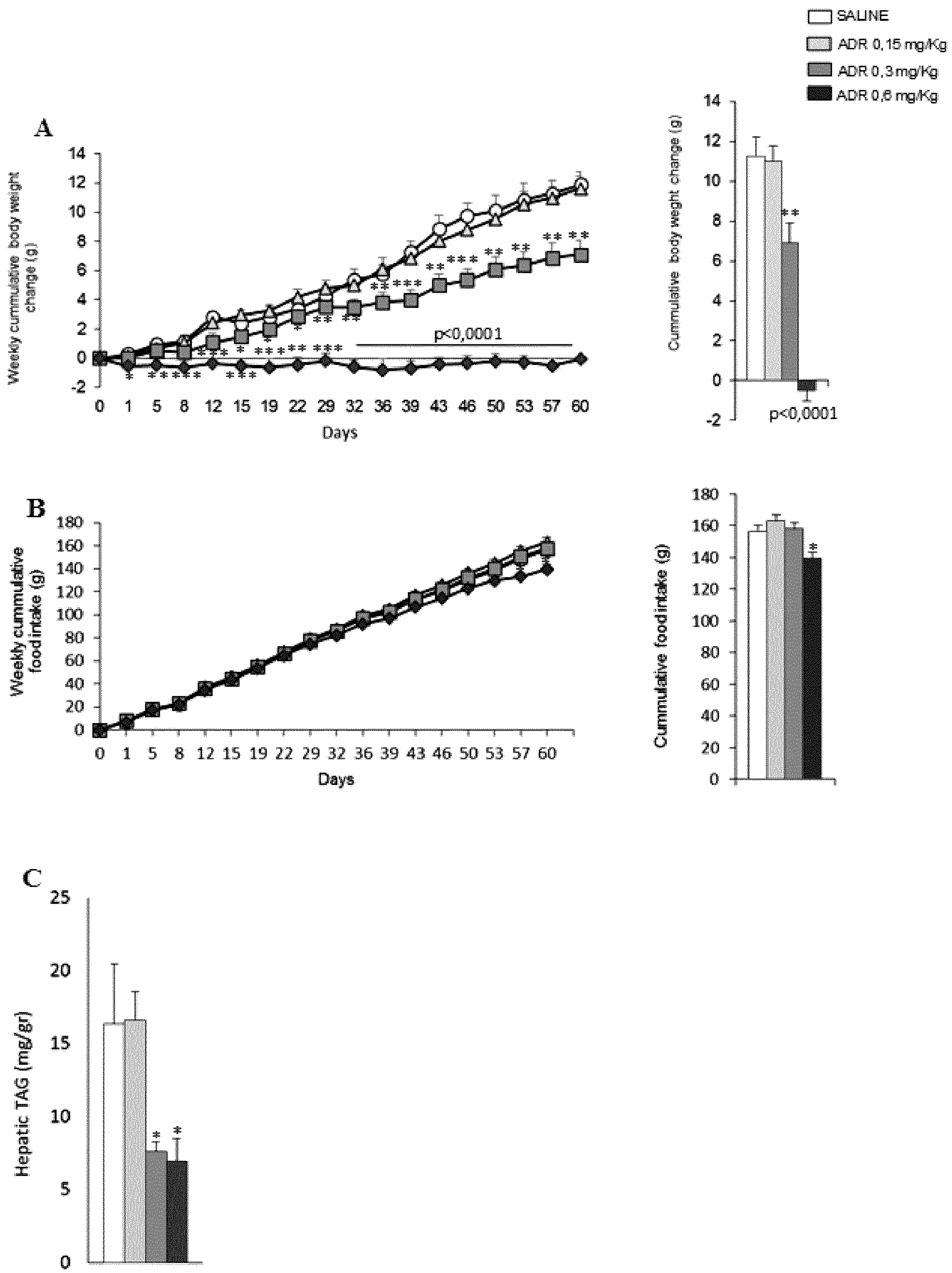
SALINE
ADR 0,15 mg/Kg
ADR 0,3 mg/Kg
ADR 0,6 mg/Kg
A
Weekly cummulative body weight change (g)
Days
p<0,0001
Cummulative body weght change (g)
p<0,0001
B
Weekly cummulative food intake (g)
Days
Cummulative food intake (g)
C
Hepatic TAG (mg/gr)

**Figure 5**

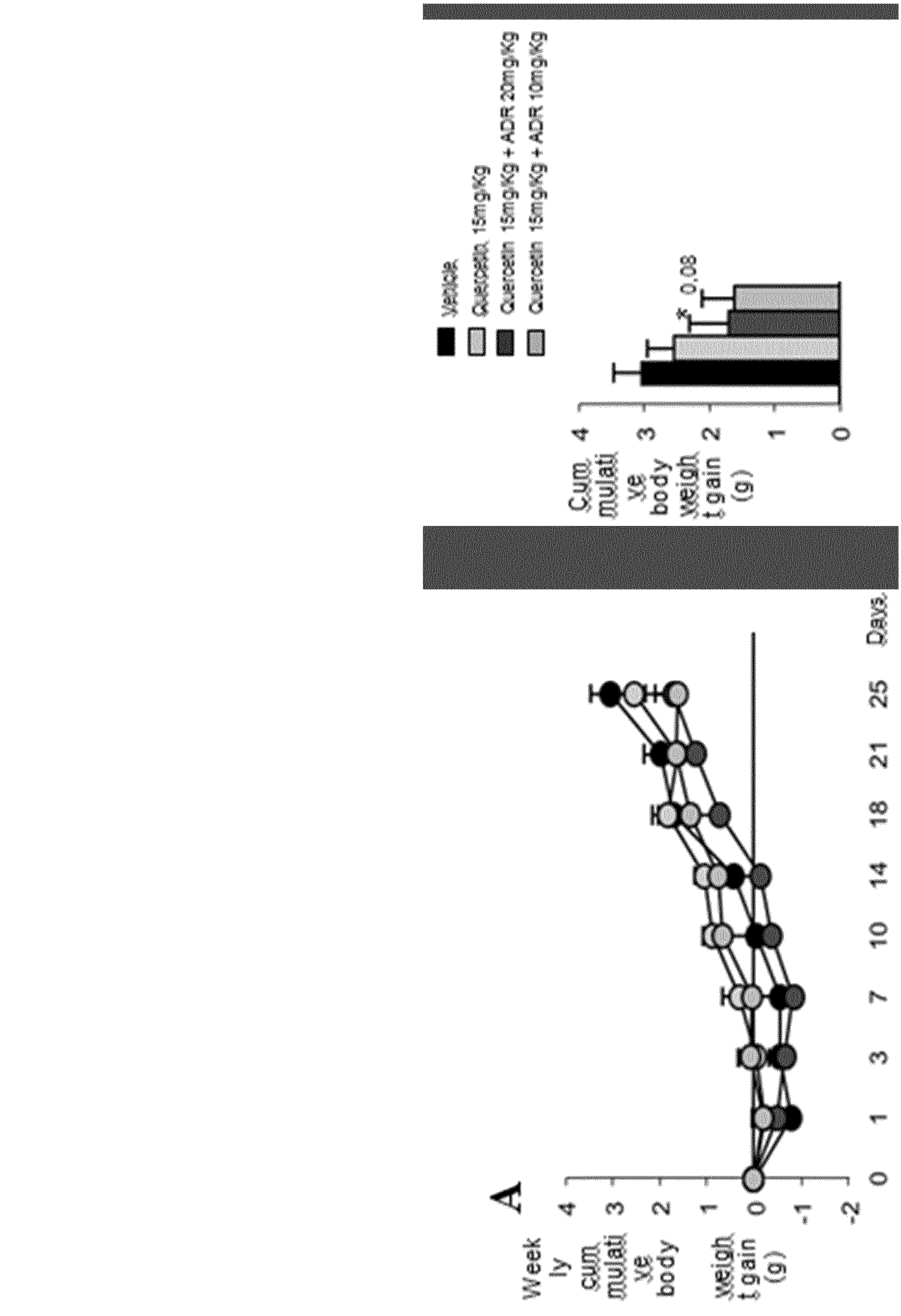
Vehicle
Quercetin 15mg/Kg
Quercetin 15mg/Kg + ADR 20mg/Kg
Quercetin 15mg/Kg + ADR 10mg/Kg
Cumulative body weight gain (g)
0,08
*
A
Weekly cumulative body weight gain (g)
Days

**Figure 5 (cont.)**

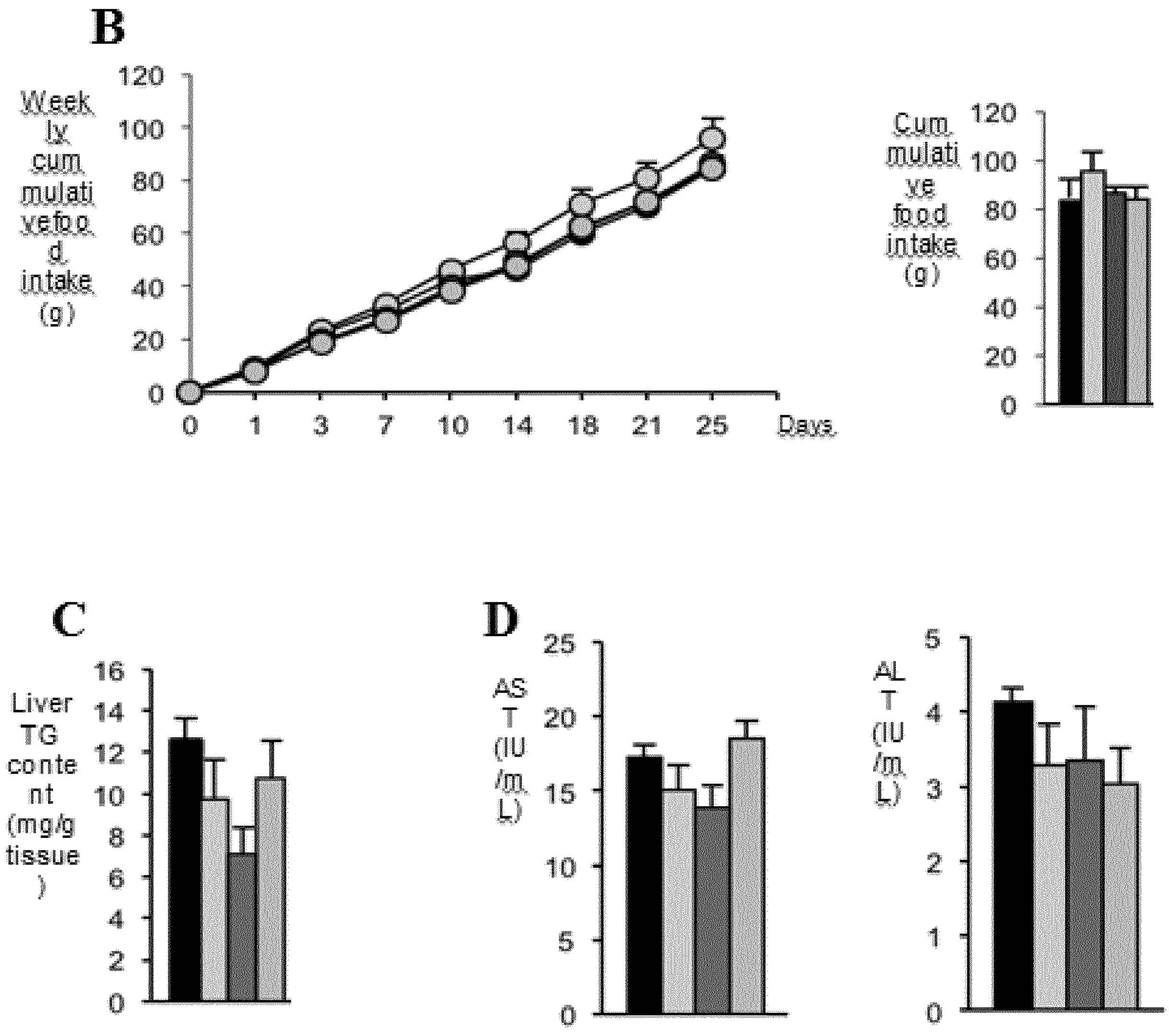

## Figure 6

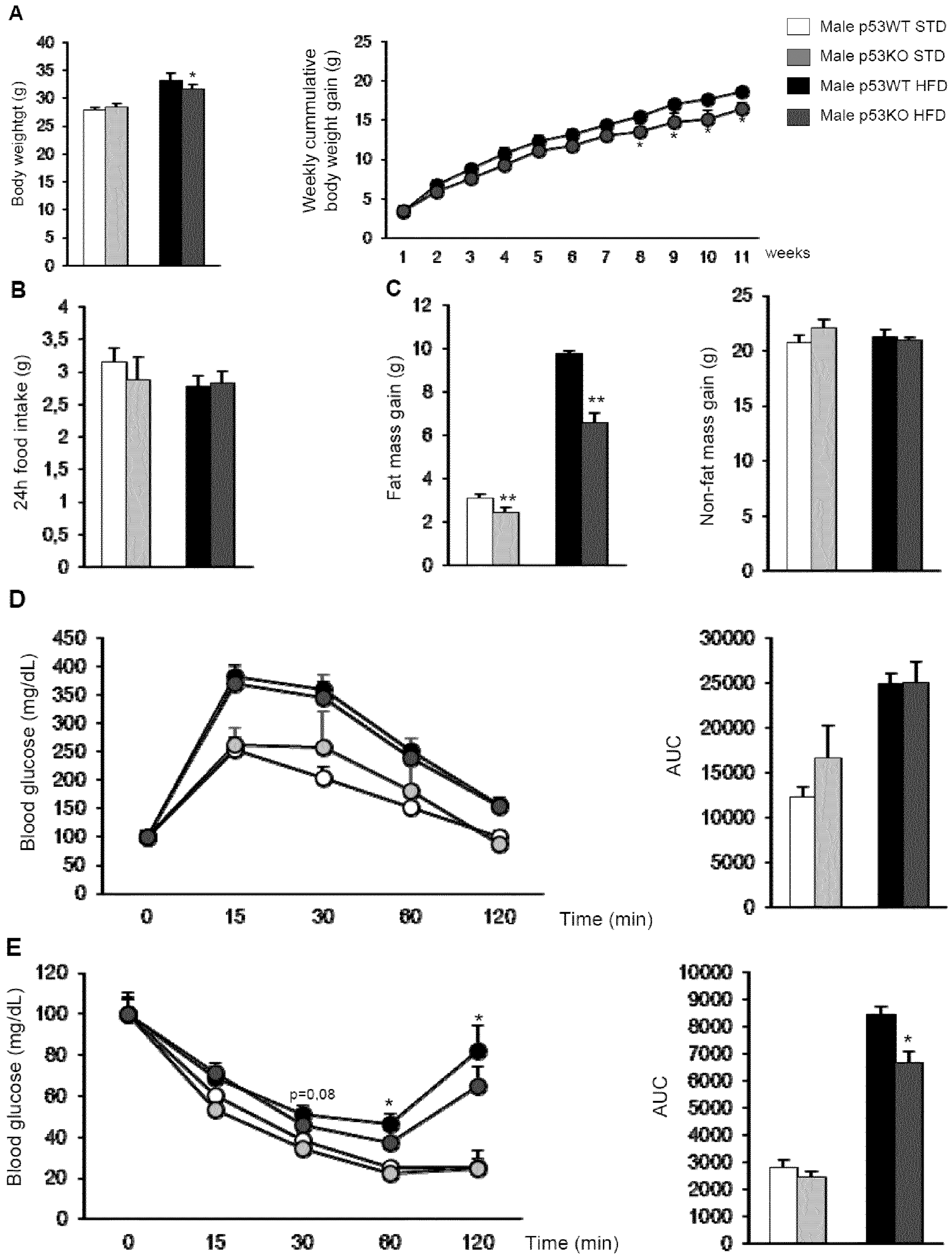
A
Body weightgt (g)
Weekly cummulative body weight gain (g)
weeks
Male p53WT STD
Male p53KO STD
Male p53WT HFD
Male p53KO HFD
B
24h food intake (g)
C
Fat mass gain (g)
Non-fat mass gain (g)
D
Blood glucose (mg/dL)
Time (min)
AUC
E
Blood glucose (mg/dL)
p=0,08
Time (min)
AUC

Figure 7

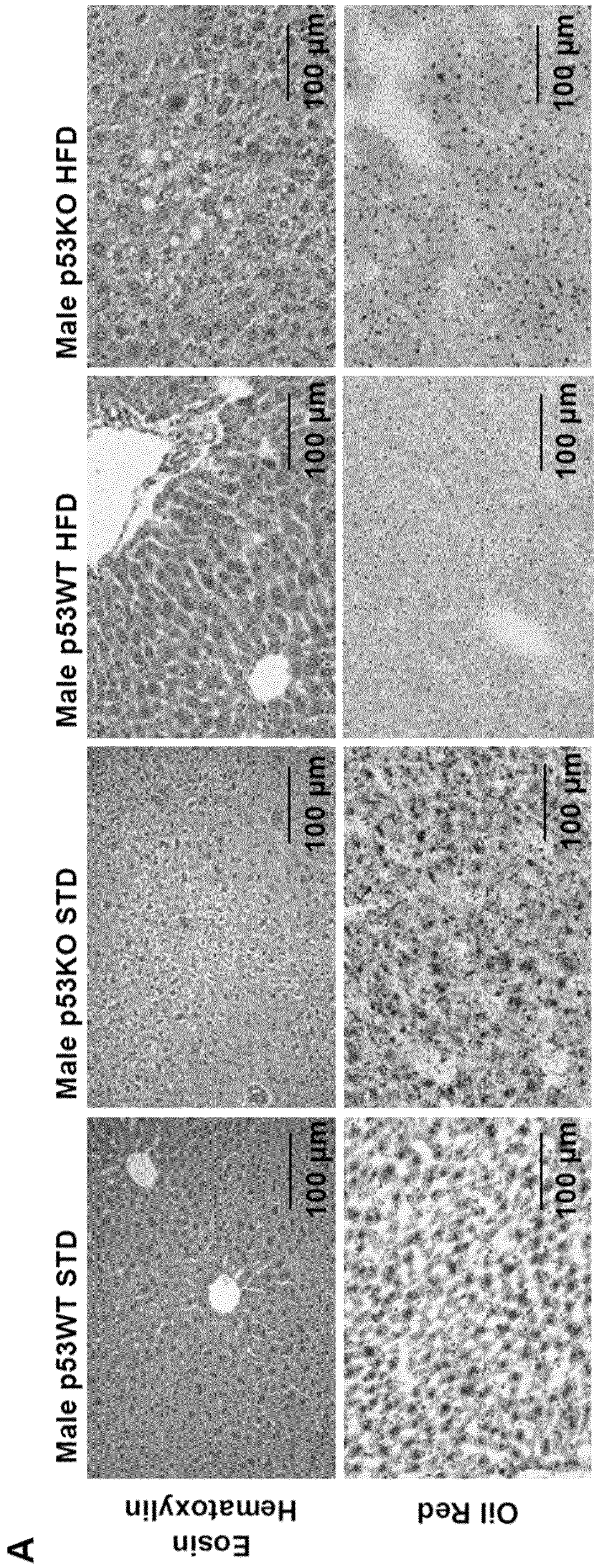
A
Hematoxylin Eosin
Oil Red
Male p53WT STD
Male p53KO STD
Male p53WT HFD
Male p53KO HFD
100 µm

**Figure 7 (cont.)**

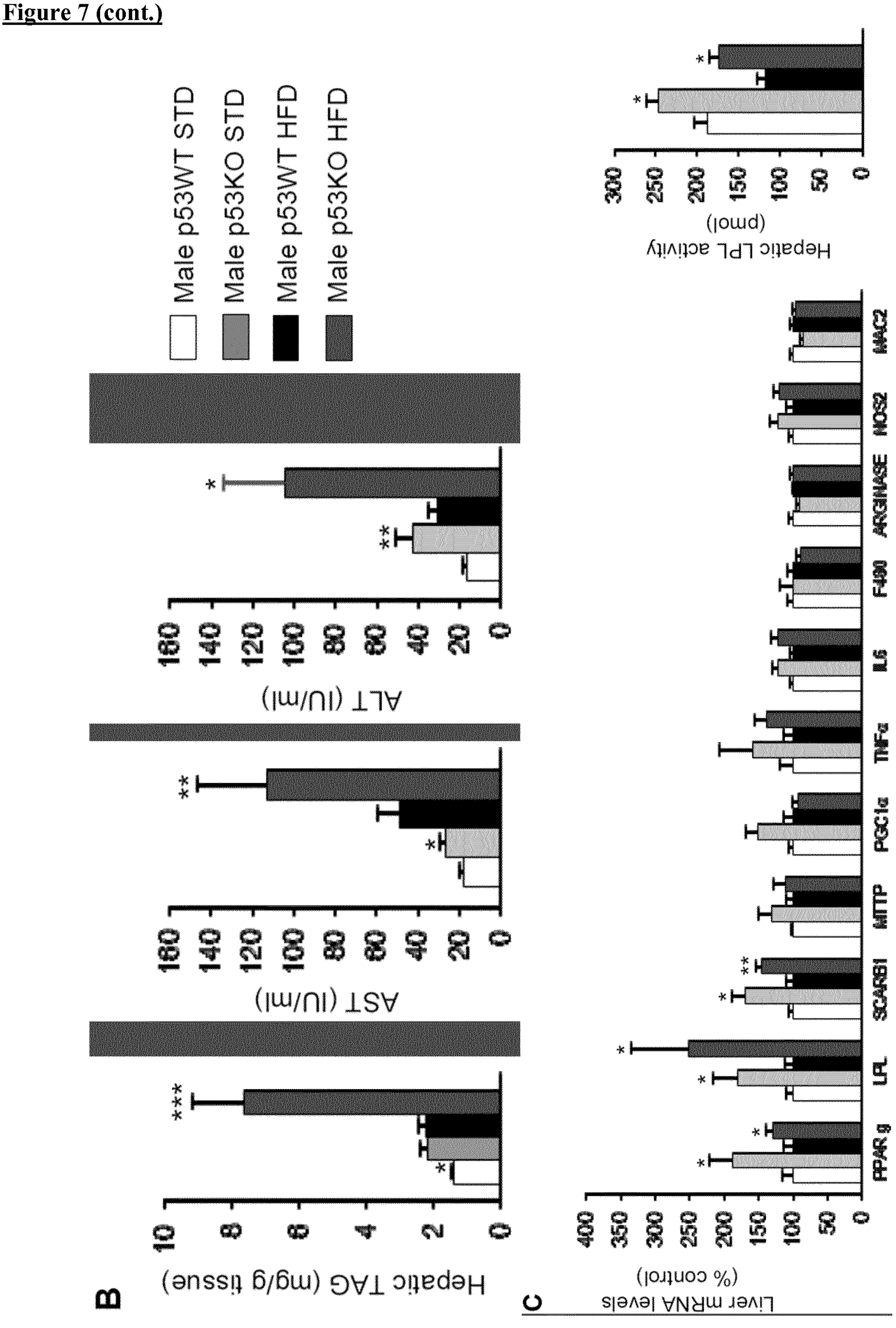

**Figure 7 (cont.)**

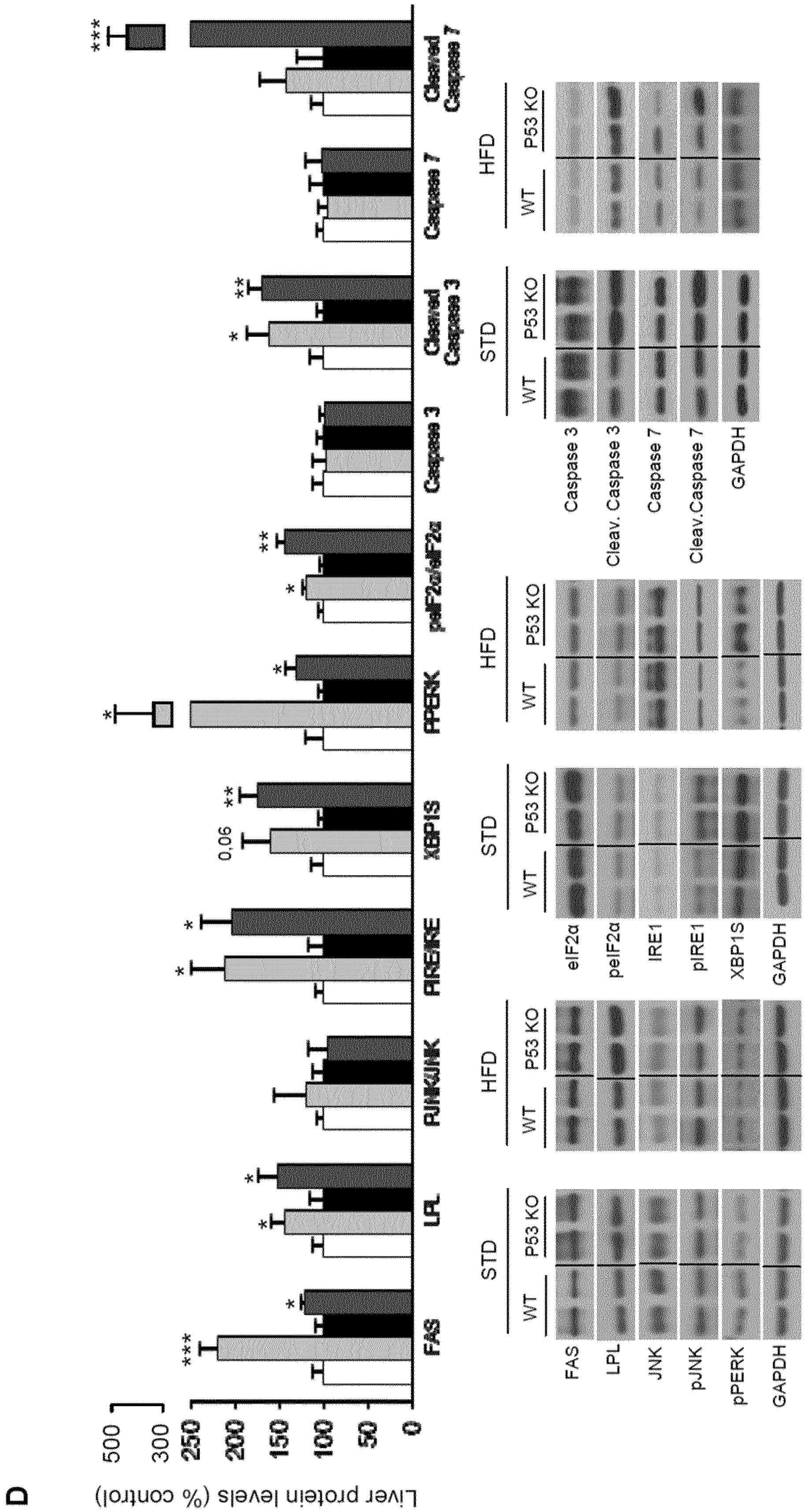

**Figure 8**

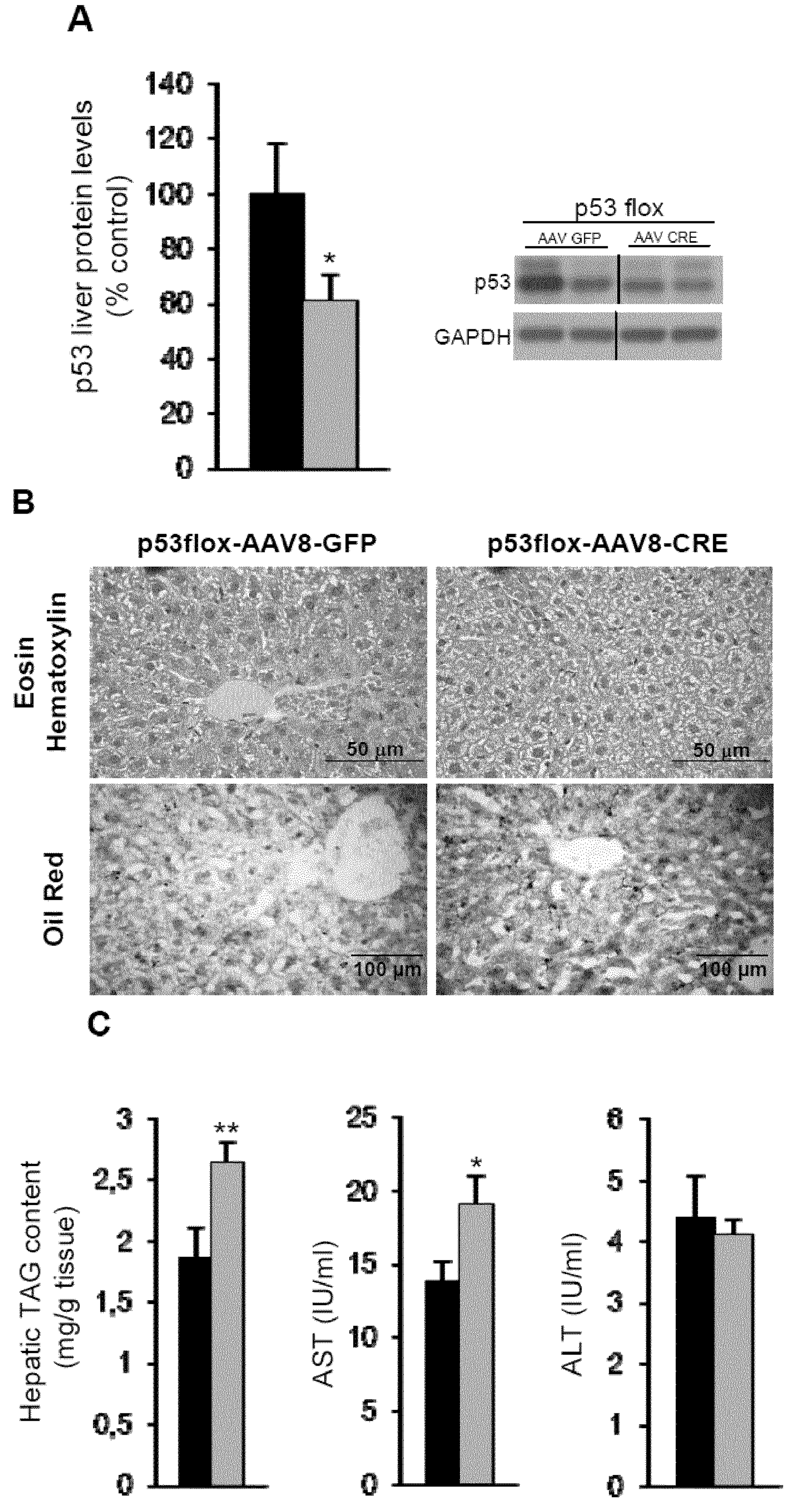
A
p53 liver protein levels (% control)
140
120
100
80
60
40
20
0
*
p53 flox
AAV GFP
AAV CRE
p53
GAPDH
B
p53flox-AAV8-GFP
p53flox-AAV8-CRE
Eosin Hematoxylin
Oil Red
50 μm
100 μm
C
Hepatic TAG content (mg/g tissue)
3
2,5
2
1,5
1
0,5
0
**
AST (IU/ml)
25
20
15
10
5
0
*
ALT (IU/ml)
6
5
4
3
2
1
0

**Figure 8 (cont.)**

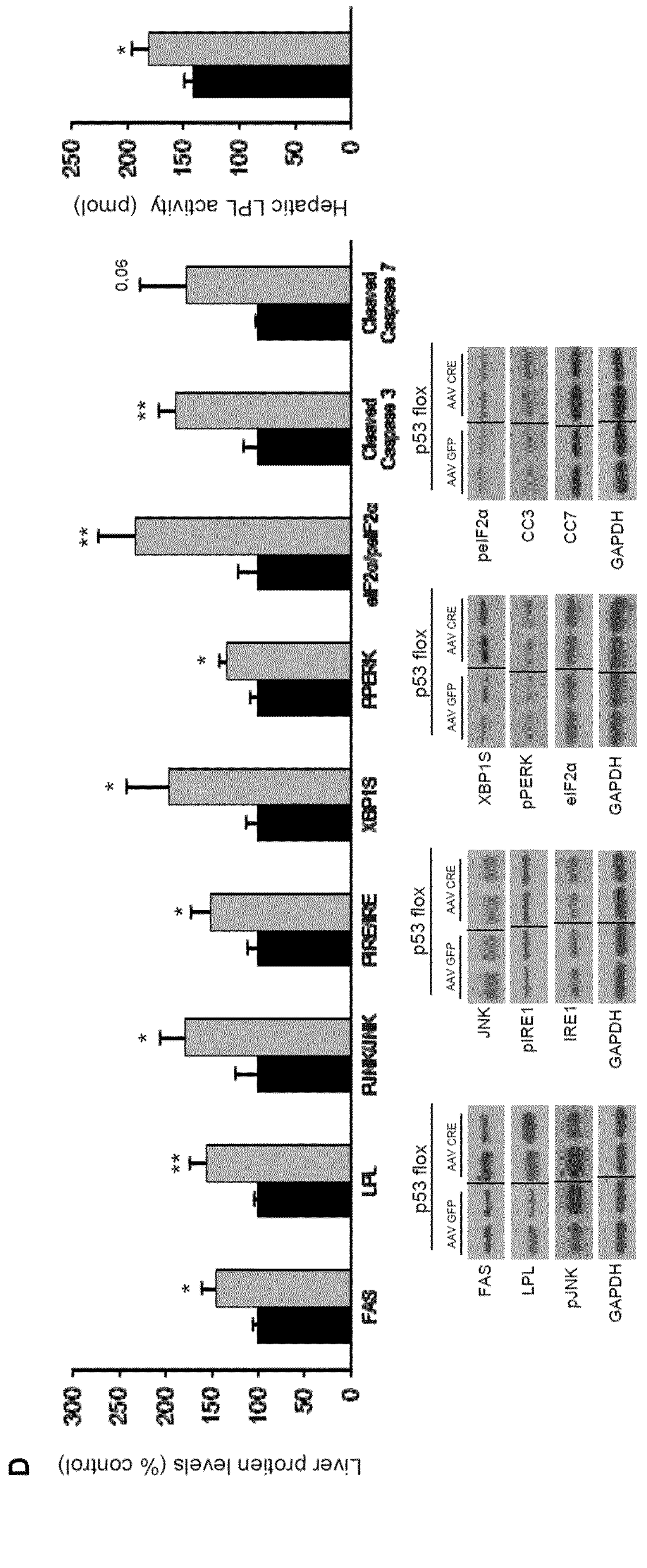

**Figure 8 (cont.)**

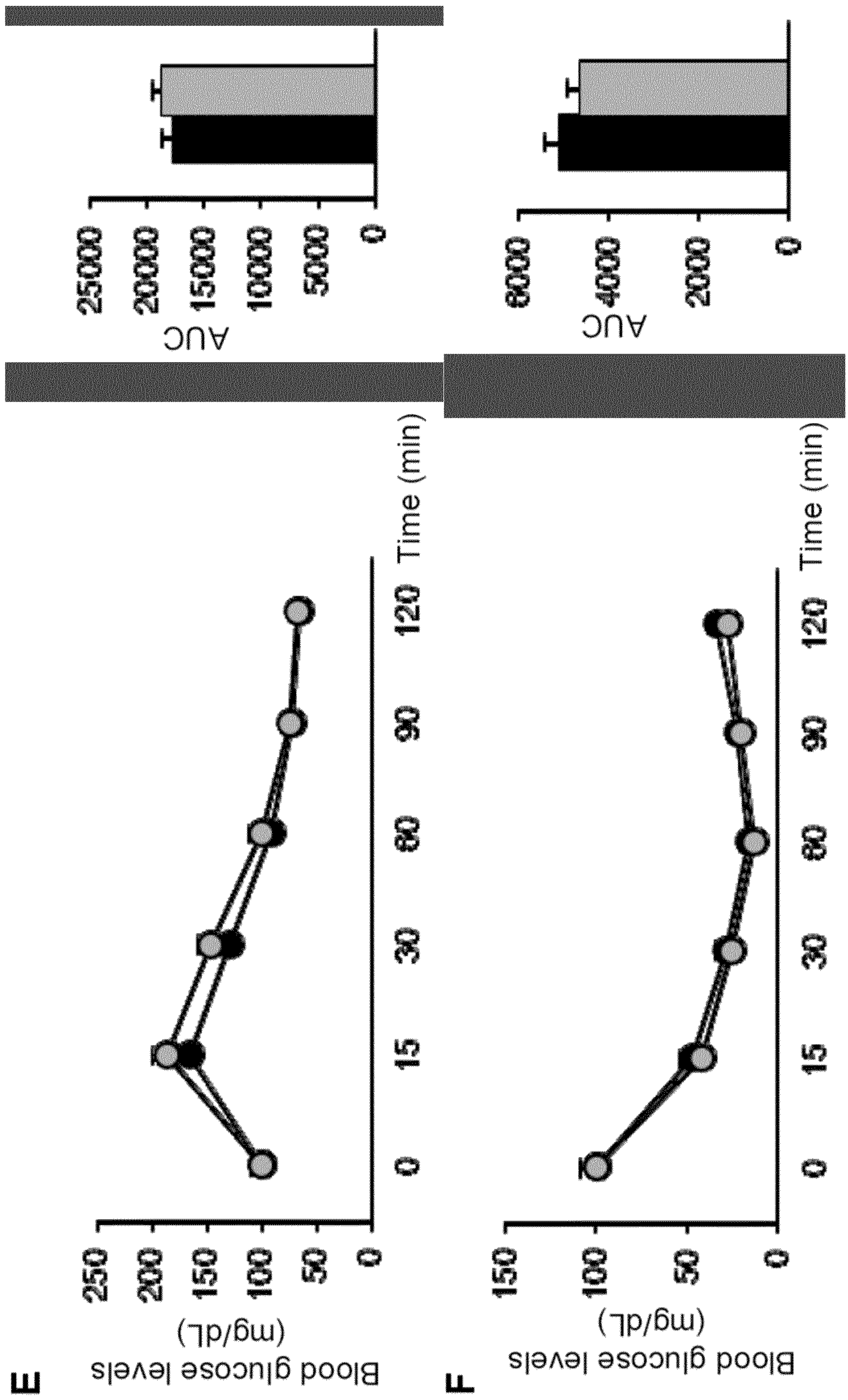

Figure 9

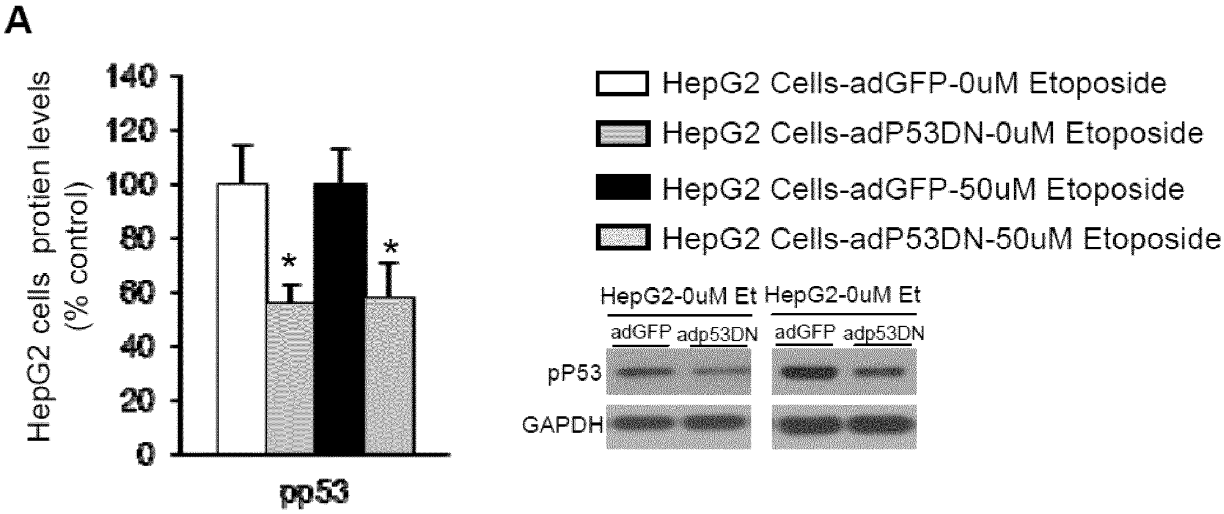
A
HepG2 cells protien levels (% control)
140
120
100
80
60
40
20
0
pp53
HepG2 Cells-adGFP-0uM Etoposide
HepG2 Cells-adP53DN-0uM Etoposide
HepG2 Cells-adGFP-50uM Etoposide
HepG2 Cells-adP53DN-50uM Etoposide
HepG2-0uM Et
HepG2-0uM Et
adGFP
adp53DN
pP53
GAPDH

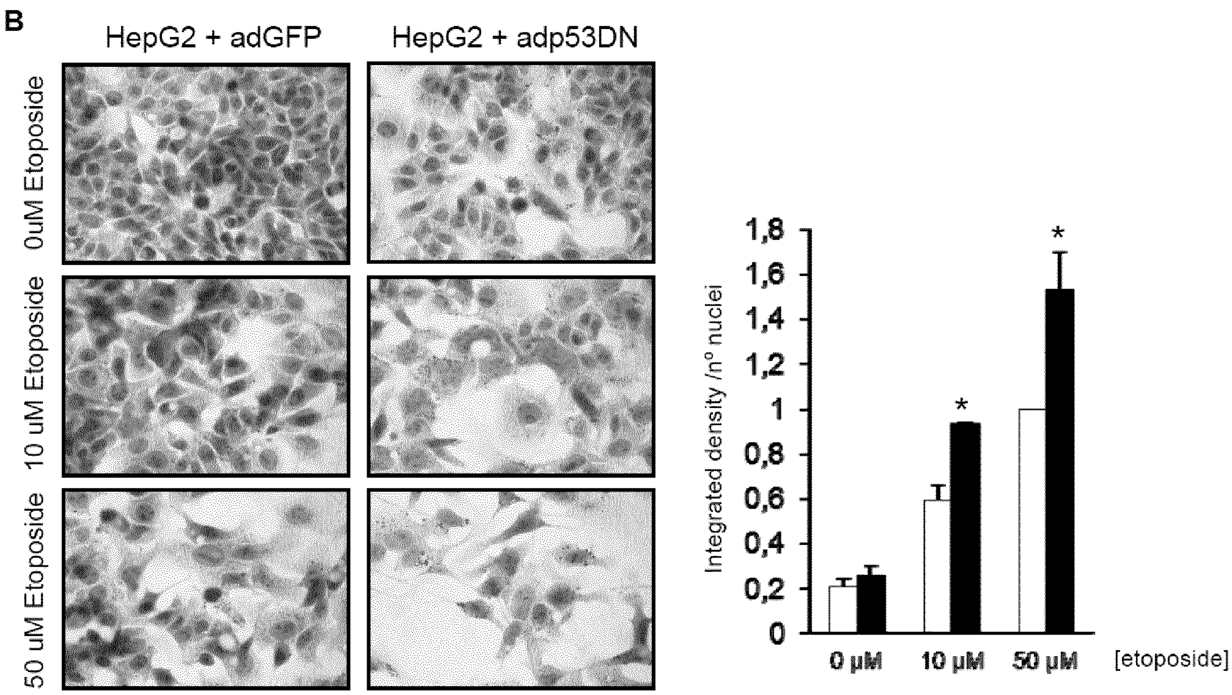
B
HepG2 + adGFP
HepG2 + adp53DN
0uM Etoposide
10 uM Etoposide
50 uM Etoposide
Integrated density /n° nuclei
1,8
1,6
1,4
1,2
1
0,8
0,6
0,4
0,2
0
0 µM
10 µM
50 µM
[etoposide]

**Figure 9 (cont.)**

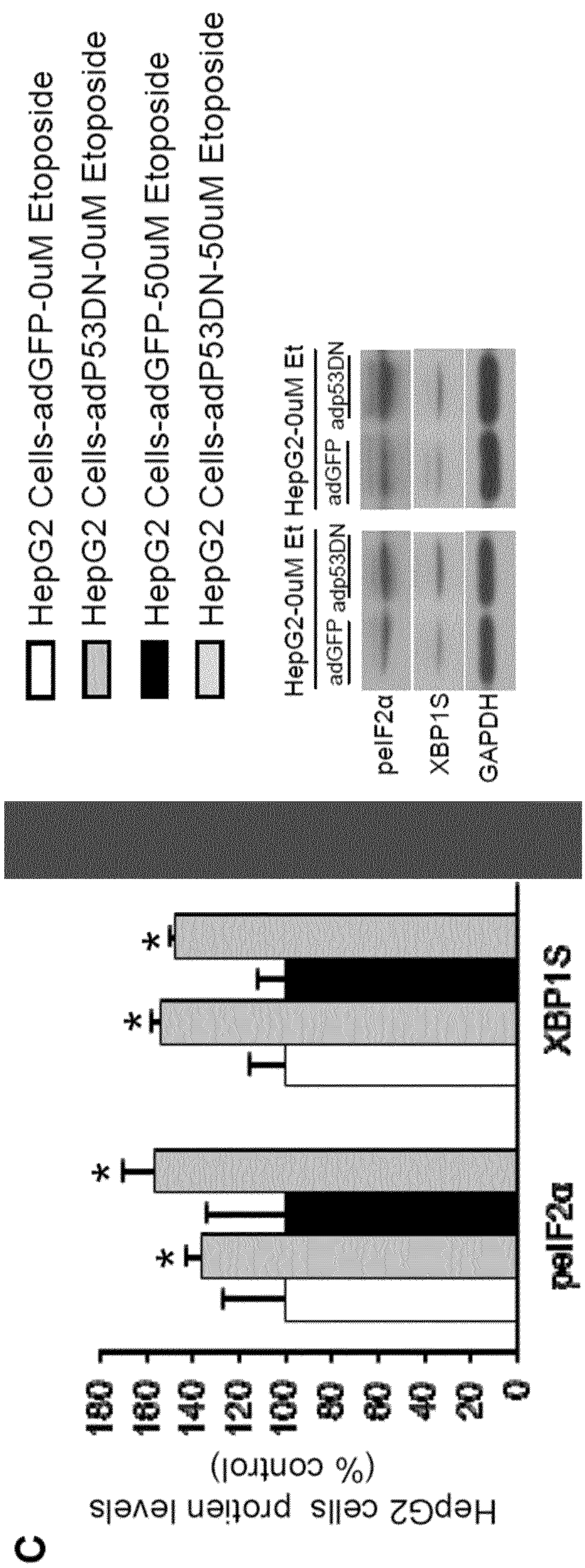

**Figure 10**

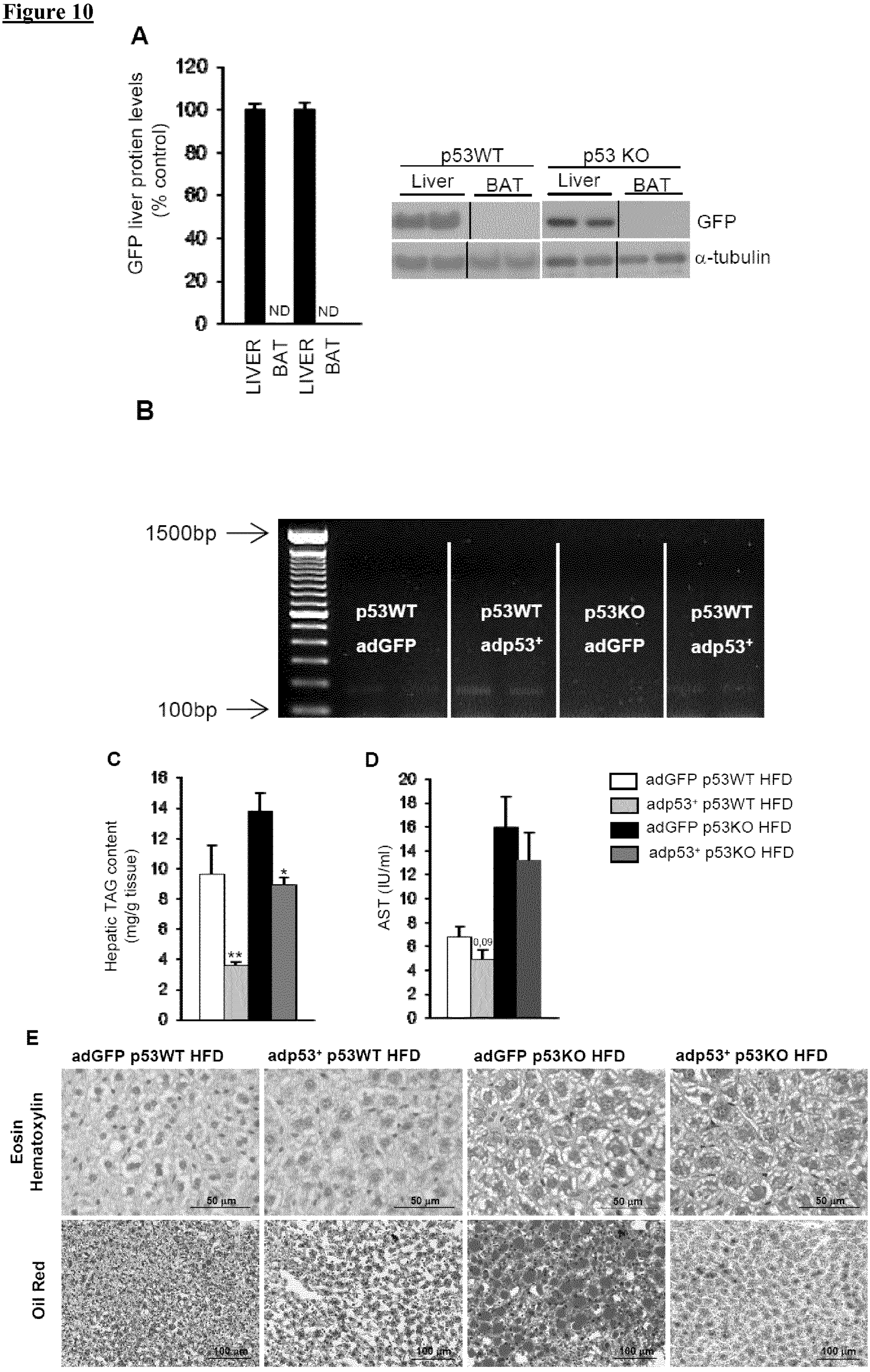
A
GFP liver protien levels (% control)
120
100
80
60
40
20
0
ND
ND
LIVER
BAT
LIVER
BAT
p53WT
p53 KO
Liver
BAT
Liver
BAT
GFP
α-tubulin
B
1500bp
100bp
p53WT adGFP
p53WT adp53+
p53KO adGFP
p53WT adp53+
C
Hepatic TAG content (mg/g tissue)
16
14
12
10
8
6
4
2
0
**
*
D
AST (IU/ml)
20
18
16
14
12
10
8
6
4
2
0
0,09
adGFP p53WT HFD
adp53+ p53WT HFD
adGFP p53KO HFD
adp53+ p53KO HFD
E
adGFP p53WT HFD
adp53+ p53WT HFD
adGFP p53KO HFD
adp53+ p53KO HFD
Hematoxylin Eosin
Oil Red
50 µm
100 µm

Figure 10 (cont.)

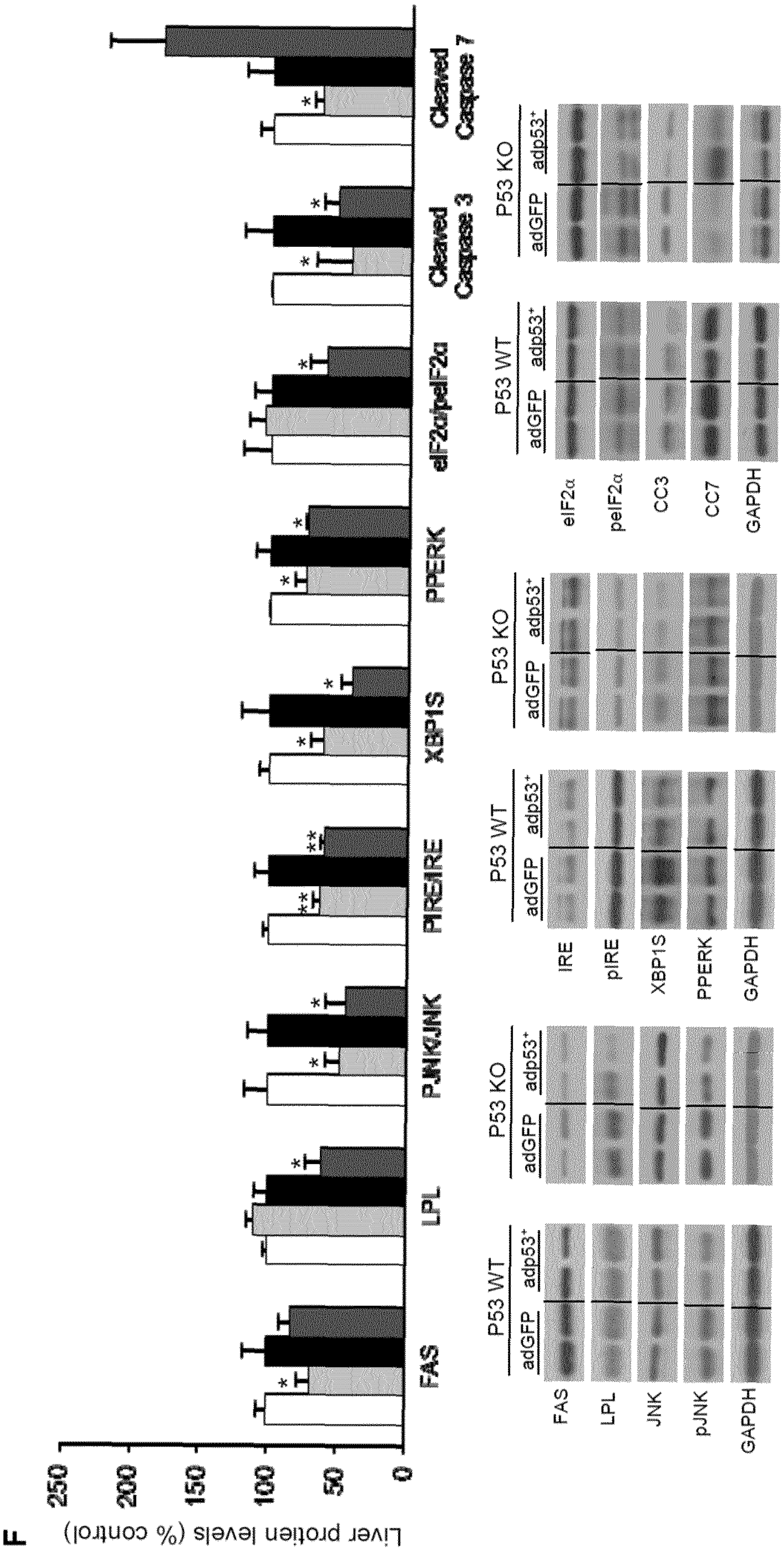

**Figure 11**

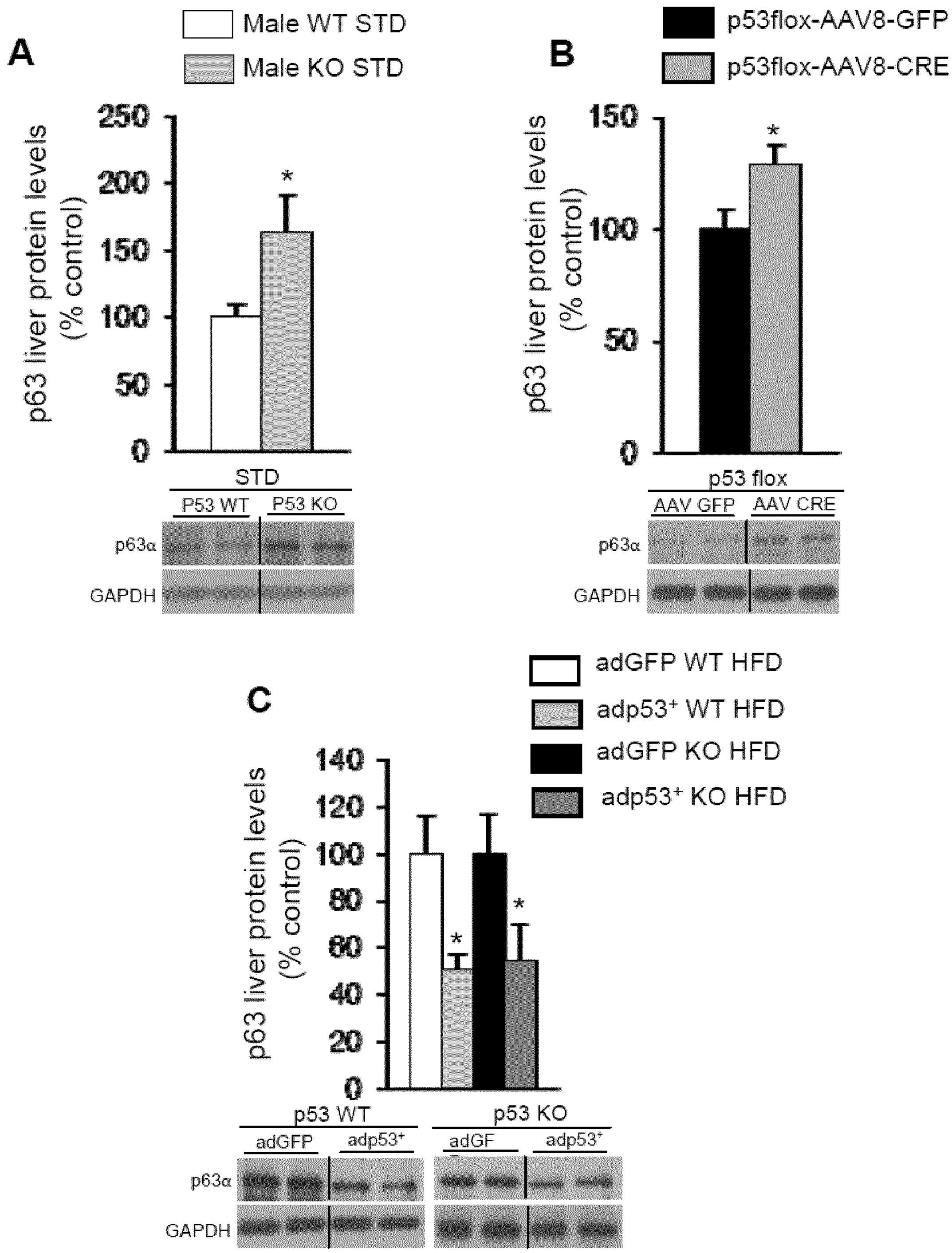
A
Male WT STD
Male KO STD
p63 liver protein levels (% control)
250
200
150
100
50
0
*
STD
P53 WT
P53 KO
p63α
GAPDH
B
p53flox-AAV8-GFP
p53flox-AAV8-CRE
p63 liver protein levels (% control)
150
100
50
0
*
p53 flox
AAV GFP
AAV CRE
p63α
GAPDH
C
adGFP WT HFD
adp53+ WT HFD
adGFP KO HFD
adp53+ KO HFD
p63 liver protein levels (% control)
140
120
100
80
60
40
20
0
*
*
p53 WT
p53 KO
adGFP
adp53+
adGF
adp53+
p63α
GAPDH

Figure 11 (cont.)

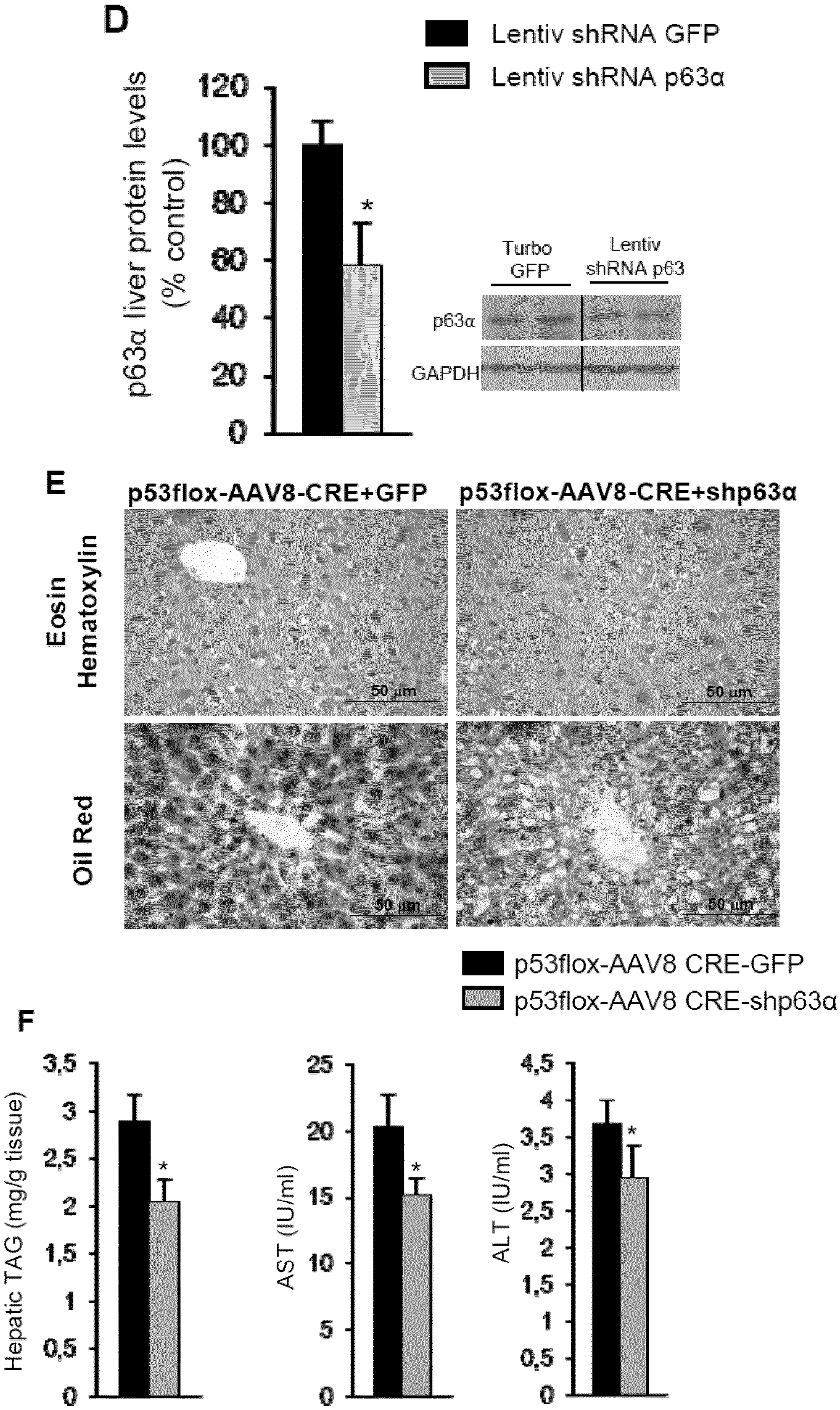

**Figure 11 (cont.)**

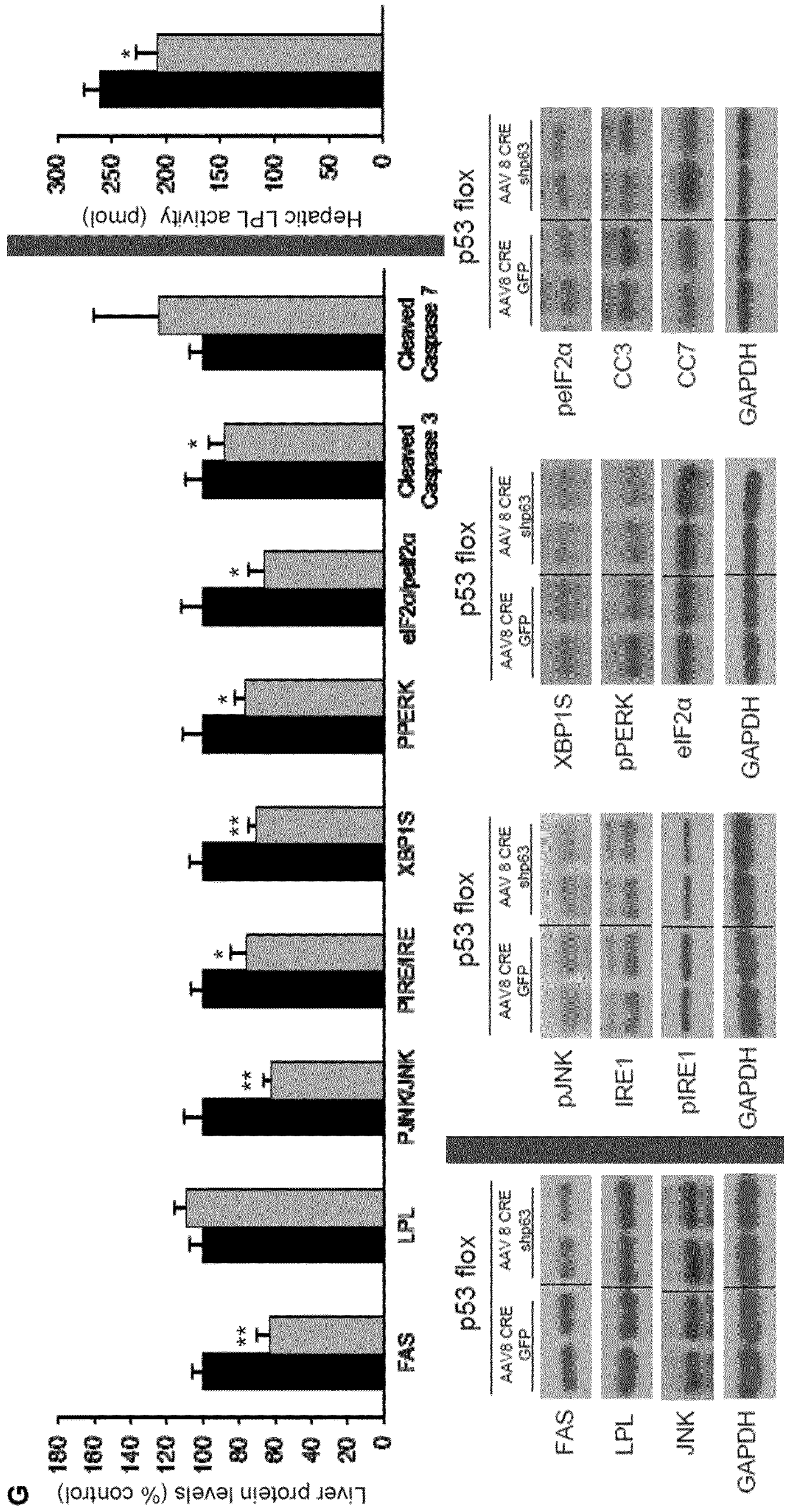

Figura 12

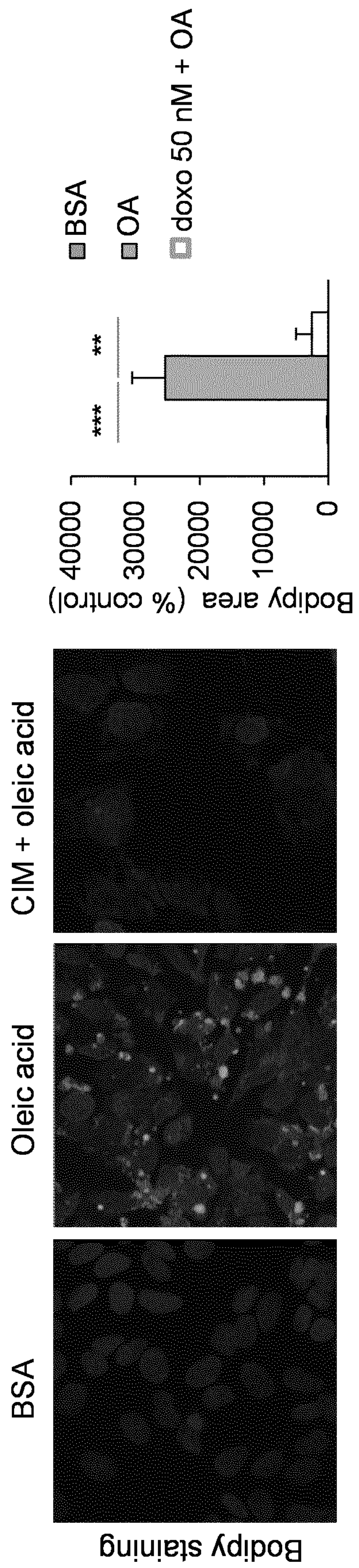
BSA
Oleic acid
CIM + oleic acid
Bodipy staining
Bodipy area (% control)
40000
30000
20000
10000
0
***
**
BSA
OA
doxo 50 nM + OA

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- **CASTRO RUI E et al.** TI-miR-34a/SIRT1/p53 is suppressed by ursodeoxycholic acid in the rat liver and activated by disease severity in human non-alcoholic fatty liver disease.AU. *J. OF HEPATOLOGY,* 15 September 1981, vol. 28
- **TOMITA et al.** p53/p66hc-mediated signalling contributes to the progression of non-alcoholic steatohepatitis in humans and mice. *Journal of Hepatology,* 01 January 2012, vol. 57, 837-843
- **REAGAN-SHAW et al.** Dose translation from animal to human studies revisited. *The FASEB Journal,* 2007, vol. 22, 659-661